# EUROPEAN PATENT SPECIFICATION

(11) **EP 2 502 496 B1**
(45) Date of publication and mention of the grant of the patent: **18.03.2015**
(21) Application number: 10830010.4
(22) Date of filing: 12.11.2010
(51) Int. Cl.: A01N 65/36, A01G 7/06, A01G 17/00, C07K 14/415, C12N 15/09

(54) **FLOWERING INDUCER**
BLÜTENSTIMULATOR
INDUCTEUR DE FLORAISON

(30) Priority: 16.11.2009 JP 2009260847
(43) Date of publication of application: 26.09.2012
(73) Proprietor: Saga University, Saga-shi, Saga 840-8502 (JP)
(72) Inventor: MATSUMOTO Ryoji, Saga-shi Saga 840-8502 (JP); NAGANO Yukio, Saga-shi Saga 840-8502 (JP)
(74) Representative: Woods, Geoffrey Corlett
(86) International application number: PCT/JP2010/070181
(87) International publication number: WO 2011/059051

(56) References cited:
- EP-A1- 2 052 599
- JP-A- 6 233 685
- JP-A- H11 512 289
- FUMIE NISHIKAWA ET AL: "Increased CiFT abundance in the stem correlates with floral induction by low temperature in Satsuma mandarin (Citrus unshiu Marc.)", JOURNAL OF EXPERIMENTAL BOTANY, OXFORD UNIVERSITY PRESS, GB, vol. 58, no. 14, 1 January 2007 (2007-01-01), pages 3915-3927, XP008156620, ISSN: 0022-0957
- TOMOKO ENDO ET AL: "Ectopic Expression of an FT Homolog from Citrus Confers an Early Flowering Phenotype on Trifoliate Orange (Poncirus trifoliata L. Raf.)", TRANSGENIC RESEARCH, KLUWER ACADEMIC PUBLISHERS-PLENUM PUBLISHERS, NE, vol. 14, no. 5, 1 October 2005 (2005-10-01), pages 703-712, XP019269469, ISSN: 1573-9368
- FUMIE NISHIKAWA ET AL.: 'Increased CiFT abundance in the stem correlates with floral induction by low temperature in Satsuma mandarin (Citrus unshiu Marc.)' JOURNAL OF EXPERIMENTAL BOTANY vol. 58, no. 14, 2007, pages 3915 - 3927, XP008156620
- YASUSHI KOBAYASHI ET AL.: 'A Pair of Related Genes with Antagonistic Roles in Mediating Flowering Signals' SCIENCE vol. 286, no. 5446, 1999, pages 1960 - 1962, XP002908230

## Description

### TECHNICAL FIELD

The present invention relates to a flowering inducer and the like.

### BACKGROUND ART

Fruit trees such as citrus do not bear flowers after a certain number of years from germination. While leaves and stalks are referred to as vegetative organs in contrast to reproductive organs such as flowers, fruit trees during the non-flowering period continue vegetative growth where only vegetative organs arises. The period during which this vegetative growth lasts is called the juvenile stage. For citrus hybrid seedlings, the juvenile stage lasts for 8-12 years. The juvenile stage is followed by the mature stage. During this mature stage, the fruit trees undergo reproduction growth (a term in contrast to vegetative growth; the growth process related to reproduction such as flowering and bearing is referred to as reproduction growth), during which flowering arises.

For fruit tree cultivation, grafting is often carried out. One of the reasons for performing grafting is to solve the problem of long period of the juvenile stage of the fruit trees (Non-patent Document 1: Nobuhito Mitani, Ryoji Matsumoto, Terutaka Yoshioka, and Takeshi Kuniga (2008) "Citrus hybrid seedlings reduce initial time to flower when grafted onto shiikuwasha rootstock" Scientia Horticulturae 116, 452-455). In the case of *Citrus unshiu,* a variety of citrus, a scion of *Citrus unshiu* at the mature stage is often grafted to a rootstock of trifoliate orange. In this case, the scion is at the mature stage. Accordingly, the scion inherently has a floral bud-forming ability. The scion, however, only undergoes vegetative growth for a while after grafting and does not form a floral bud. This vegetative growth phase of this part of the scion (the part of the grafted seedling) is referred to as a nutritional mature stage. The nutritional mature stage generally lasts for about four years. However, in view of reduction in the cost of cultivation, early investment recovery and efficiency in variety improvement, development of a method for shortening the period of nutritional mature stage has been sought.

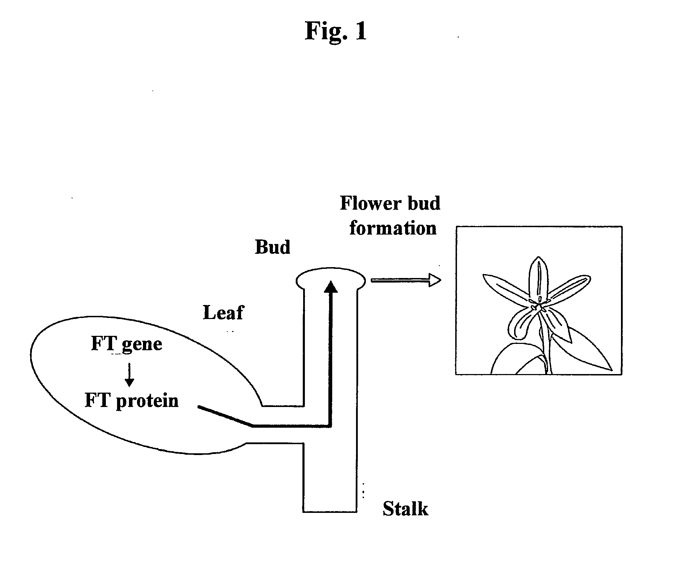

Now, as a gene involved in flowering, the gene of "Flowering Locus T" (hereinafter, referred to as "FT gene") is known. A protein encoded by this FT gene is called FT protein. As shown in Figure 1, FT protein is already known to be synthesized in the leaves and translocated to the shoot according to environmental change such as the day length, where it induces floral bud formation (Non-patent Document 2: Laurent Corbesier, Coral Vincent, Seonghoe Jang, Fabio Fornara, Qingzhi Fan, Iain Searle, Antonis Giakountis, Sara Farrona, Lionel Gissot, Colin Turnbull, and George Coupland (2007) "FT Protein Movement Contributes to Long-Distance Signaling in Floral Induction of Arabidopsis" Science 316, 1030-1033). Therefore, while a substance that is synthesized in the leaves and then translocated to the shoot to induce floral bud formation is referred to as a flowering hormone, FT protein is considered to be the principal body of this flowering hormone.

Studies for accelerating the beginning of the flowering period of *Arabidopsis* have been in progress using FT gene of *Arabidopsis*. For example, according to the method described in Patent Document 1 (Japanese Unexamined Patent Application Publication (Translation of PCT Application) No. 2002-511270), in order to express FT protein at a high level in a plant body, a DNA fragment containing FT gene linked behind cauliflower mosaic virus 35S promoter is introduced into *Arabidopsis* to produce recombinant *Arabidopsis.* As a result, recombinant *Arabidopsis* with earlier flowering period is obtained.

Similar study was also performed for citrus (Non-patent Document 3: Tomoko Endo, Takehiko Shimada, Hiroshi Fujii, Yasushi Kobayashi, Takashi Araki, and Mitsuo Omura (2005) "Ectopic expression of an FT homolog from citrus confers an early flowering phenotype on trifoliate orange (Poncirus trifoliata L. Raf.)" Transgenic Research 14, 703-712.). In this study, *Citrus unshiu* Marc.-derived CiFT gene that is highly homologous with *Arabidopsis* FT gene is used. According to this method, a DNA fragment containing CiFT gene linked behind cauliflower mosaic virus 35S promoter is introduced into trifoliate orange (Poncirus trifoliata) to generate recombinant trifoliate orange. As a result, the beginning of the flowering period of trifoliate orange is accelerated. More specifically, flowering was observed in a minimum of about half year after the appearance of epicotyl.

In all of these prior arts, FT protein (a protein highly homologous with *Arabidopsis* FT protein, such as CiFT protein above, is also simply referred to herein as "FT protein") is highly expressed in a plant body using a recombinant plant. However, methods that employ a recombinant plant have several concerns.

One of the concerns is that the resulting plant with earlier flowering is a recombinant plant. Since recombinant plants are associated with many legal restrictions, it would take a fair amount of time before these recombinant plants can be cultivated in a farm field. In addition, there is a situation where consumers' understanding for recombinant plants has not been increased.

The other concern is that a floral bud may be formed in an extremely young plant (Non-patent Document 3: Tomoko Endo, Takehiko Shimada, Hiroshi Fujii, Yasushi Kobayashi, Takashi Araki, and Mitsuo Omura (2005) "Ectopic expression of an FT homolog from citrus confers an early flowering phenotype on trifoliate orange (Poncirus trifoliata L. Raf.)" Transgenic Research 14, 703-712.). Once a fruit is produced, growth of the leaves and thus growth of a plant body is disturbed. Therefore, since too early floral bud formation lay a heavy burden on a young plant body, it may not always be desirable for the growth of the plant as well as for the growth of the fruit.

It has also been reported that increased CiFT abundance in the stem correlates with floral induction by low temperature in Satsuma mandarin (Citrus unshiu Marc.) (Non-patent Document 4: Fumie Nishikawa, Tomoko Endo, Takehiko Shimada, Hiroshi Fujii, Tokurou Shimizu, Mitsuo Omura and Yoshinori Ikoma "Increased CiFT abundance in the stem correlates with floral induction by low temperature in Satsuma mandarin (Citrus unshiu Marc.)" J. Experimental Botany vol. 58, no. 14, 3915-3927, 2007).

### PRIOR ART DOCUMENTS

### [Patent Document]

[Patent Document 1]
Japanese Unexamined Patent Application Publication (Translation of PCT Application) No. 2002-511270

### [Non-patent Documents]

[Non-patent Document 1]
   Nobuhito Mitani, Ryoji Matsumoto, Terutaka Yoshioka, and Takeshi Kuniga (2008) "Citrus hybrid seedlings reduce initial time to flower when grafted onto shiikuwasha rootstock" Scientia Horticulturae 116, 452-455.
[Non-patent Document 2]
   Laurent Corbesier, Coral Vincent, Seonghoe Jang, Fabio Fornara, Qingzhi Fan, Iain Searle, Antonis Giakountis, Sara Farrona, Lionel Gissot, Colin Turnbull, and George Coupland (2007) "FT Protein Movement Contributes to Long-Distance Signaling in Floral Induction of Arabidopsis" Science 316, 1030-1033.
[Non-patent Document 3]
   Tomoko Endo, Takehiko Shimada, Hiroshi Fujii, Yasushi Kobayashi, Takashi Araki, and Mitsuo Omura (2005) "Ectopic expression of an FT homolog from citrus confers an early flowering phenotype on trifoliate orange (Poncirus trifoliata L. Raf.)" Transgenic Research 14, 703-712.
[Non-patent Document 4]
   Fumie Nishikawa, Tomoko Endo, Takehiko Shimada, Hiroshi Fujii, Tokurou Shimizu, Mitsuo Omura and Yoshinori Ikoma "Increased CiFT abundance in the stem correlates with floral induction by low temperature in Satsuma mandarin (Citrus unshiu Marc.)" J. Experimental Botany vol. 58, no. 14, 3915-3927, 2007).

### SUMMARY OF THE INVENTION

### PROBLEMS TO BE SOLVED BY THE INVENTION

Under such circumstances, a technique for inducing flowering without depending on gene recombination, namely, a flowering inducer that can be administered into a plant body from outside has been expected.

### MEANS FOR SOLVING THE PROBLEMS

In order to solve the above-described problems, the present inventors have gone through intensive investigation, and, as a result of which, found that administration of a predetermined FT protein into a plant body from outside can accelerate the beginning of the flowering period of the plant body administered and the plant body adjacent thereto without the need of generating a recombinant plant, thereby accomplishing the present invention.

Thus, the present invention provides:
[1] A method for inducing flowering comprising the step of injecting into a plant body
   (i) any one of proteins (A)-(C) below:
      (A) a purified protein comprising the amino acid sequence represented by SEQ ID NO:2;
      (B) a purified protein comprising an amino acid sequence having deletion, substitution and/or addition of one or several amino acids in the amino acid sequence represented by SEQ ID NO:2 and having a flowering-inducing activity; and
      (C) a purified protein comprising an amino acid sequence having an identity of 60% or more with the amino acid sequence represented by SEQ ID NO:2 and having a flowering-inducing activity; or
   (ii) a purified protein encoded by a gene comprising any one of DNAs (a)-(c) below:
      (a) DNA consisting of the nucleotide sequence represented by SEQ ID NO:1;
      (b) DNA that hybridizes with DNA consisting of a nucleotide sequence complementary to the nucleotide sequence represented by SEQ ID NO:1 under stringent conditions and that codes for a protein having a flowering-inducing activity; and
      (c) DNA comprising a nucleotide sequence having an identity of 60% or more with the nucleotide sequence represented by SEQ ID NO:1 and coding for a protein having a flowering-inducing activity.
[2] The method according to [1], wherein the said purified protein (C) comprises an amino acid sequence having a said sequence identity of 95% or more.
[3] The method according to [1], wherein the said purified protein (ii) is encoded by a gene comprising a said DNA (c) which comprises a nucleotide sequence having a said sequence identity of 95% or more.
[4] The method according to [1], wherein the protein injected into the plant body is the purified protein consisting of the amino acid sequence represented by SEQ ID NO:4.
[5] The method according to [1], wherein the protein injected into the plant body is the purified protein encoded by a gene comprising DNA consisting of the nucleotide sequence represented by SEQ ID NO:3.
[6] The method according to any one of [1] to [5], wherein the plant is a fruit tree.
[7] The method according [6], wherein the fruit tree is citrus.
[8] The method according to [7], wherein the citrus is Citrus unshiu.
[9] The method according to any one of [1] to [8], wherein the plant is a plant obtained by grafting a scion at the mature stage to a rootstock.
[10] The method according to any one of [1] to [9], wherein the plant treated with the protein is placed next to a plant targeted for flowering induction that has not been treated with the protein.
[11] Use of a purified protein as defined in any one of [1] to [5] for inducing flowering of a plant by injecting the said purified protein into the plant body.
[12] The use according to [11], wherein the plant is as defined in any one of [6] to [8].
[13] The use according to [1] or [12], wherein the plant is a plant obtained by grafting a scion at the mature stage to a rootstock.
[14] The use according to any one of [11] to [13], wherein the plant treated with the protein is placed next to a plant targeted for flowering induction that has not been treated with the protein.

### EFFECT OF INVENTION

The present invention provides a method of inducing flowering by injecting a flowering inducer into a plant body. A flowering inducer can be administered into a fruit tree to shorten the time before the flowering.

### BRIEF DESCRIPTION OF THE DRAWINGS

[Figure 1] A figure for illustrating a process of FT protein to induce floral bud formation.
[Figure 2] A figure for illustrating a process for cloning a PCR product into plasmid pET-28b(+).
[Figure 3] A figure showing the results from comparison among amino acid sequences of FT proteins from various plants.
[Figure 4] A figure showing that a protein for use in the present invention has been purified.
[Figure 5] A picture showing a one-year-old seedling (picture taken in April 2009).
[Figure 6] A picture showing an exemplary method for injecting a flowering inducer according to the present invention.
[Figure 7] A picture showing another exemplary method for injecting a flowering inducer according to the present invention.
[Figure 8] A picture showing Plant No.3 in Table 1 bearing flowers.
[Figure 9] A picture showing Plant No.21 in Table 2 bearing flowers.
[Figure 10] A picture showing Plant No.24 in Table 3 bearing flowers.
[Figure 11] A picture showing that a plant that is 2.9 m away from the plant treated by Injection Method 2 does not appear to bear a flower.
[Figure 12] A figure for illustrating a hypothesis that flowering is induced by a flowering pheromone.

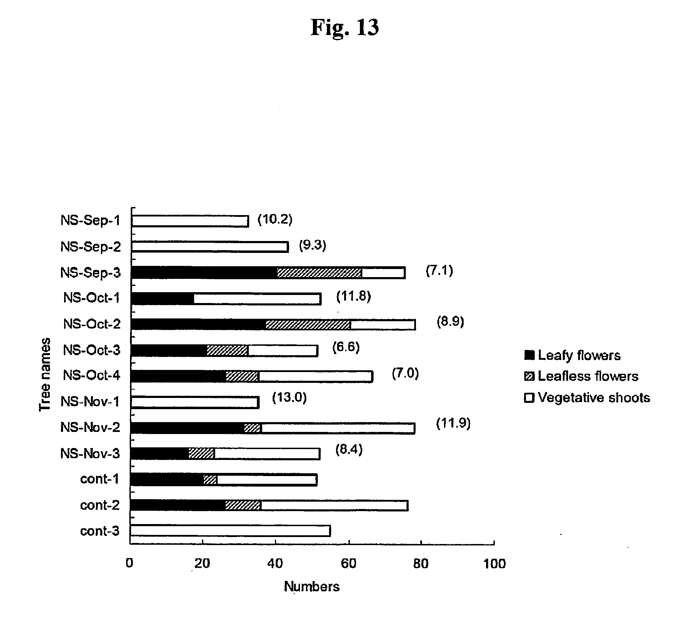

[Figure 13] A graph showing effect of top grafting of a nucellar seedling on flowering. Cont-1 to cont-3 are results of those that were 2.9 m away from the FT treatment section, which correspond to Plants Nos.37, 36 and 35 in Table 4, respectively. In the figure, "Sep", "Oct" and "Nov" following NS (nucellar seedling) represent the months of FT treatment. The numerical values in parentheses represent stem diameters of the nucellar seedlings. "Leafy flower", "leafless flower" and "vegetative shoot" refer to a leafy flower (flowering shoot), a leafless flower and a vegetative shoot (non-flowering shoot), respectively.
[Figure 14] A graph showing the relationship between the stem diameter and the number of flowers borne by the nucellar seedlings (r = -0.659 (n = 10)).
[Figure 15] A graph showing the relationship between the height and the number of flowers borne by the nucellar seedlings (r = -0.3407 (n = 10)).

### EMBODIMENTS FOR CARRYING OUT THE INVENTION

Hereinafter, the present invention will be described in detail.

### (1) Summary of the invention

The present invention uses a flowering inducer containing FT protein (hereinafter, also referred to as the protein of the present invention) as an active component. The flowering inducer is administered into a plant body from outside. So far, there is no report that administration of FT protein into a plant body from outside accelerates the beginning of the flowering period of a plant body.

The present inventors have isolated cDNA of CiFT2 gene that has high homology with *Arabidopsis* FT gene by using RNA extracted from a *Citrus unshiu* leaf as a template. cDNA of CiFT2 gene was cloned into plasmid pET-28b(+) to construct a plasmid inserted with CiFT2 gene. This plasmid was used to transform *E. coli,* thereby obtaining a transformant. The resulting transformant was used to produce CiFT2 protein.

The produced CiFT2 protein was administered to a one-year-old seedling. This one-year-old seedling had trifoliate orange as the rootstock to which *Citrus unshiu* was grafted as a scion and grown for 12 months. CiFT2 protein was administered to at the upper part of the grafted site on the main stem of this one-year-old seedling. As a result, for the CiFT2 protein-administered group, bearing of a lot of leafy flowers was observed from April to May, i.e., 5-13 months after the administration. Furthermore, bearing of many leafy flowers was also observed for the group not administered with CiFT2 protein and placed next to the CiFT2 protein-administered group. Leafy flowers are flowers with leaves, which, in comparison to leafless flowers, i.e., floral buds with flowers only, have a higher bearing rate and quality of fruits produced therefrom is often good.

### (2) Protein of the present invention

The protein of the present invention, for example, comprises the amino acid sequence represented by SEQ ID NO:2. A protein comprising the amino acid sequence represented by SEQ ID NO:2 is, for example, a protein consisting of the amino acid sequence represented by SEQ ID NO:2.

In addition, the protein of the present invention is not limited to the above-described proteins, and comprises a protein containing whole or a part of the amino acid sequence represented by SEQ ID NO:2 and having a flowering-inducing activity.

Here, a "flowering-inducing activity" refers to an activity of accelerating the beginning of the flowering period of a plant such that it begins earlier than the usual flowering period, an activity of promoting the flowering of a plant, or the like.

The protein of the present invention also comprises a protein containing amino acid sequences having homology (identity) of about 60% or more, preferably about 70% or more, more preferably about 80% or more, still more preferably about 90% or more, particularly preferably about 95% or more, yet still more preferably about 98% or more with the amino acid sequence represented by SEQ ID NO:2 and having a flowering-inducing activity.

The value of the above-mentioned identity is generally the larger the better. The identity of an amino acid sequence may be determined by using an analysis program such as BLAST (see, for example, Altzshul S. F. et al., J. Mol. Biol. 215, 403 (1990)). When using BLAST, the default parameters for each program are used.

Furthermore, the protein of the present invention also comprises a protein containing an amino acid sequence having deletion, substitution and/or addition of one or several amino acids in the amino acid sequence represented by SEQ ID NO:2, and having a flowering-inducing activity.

An amino acid sequence having a mutation of deletion, substitution and/or addition of one or several amino acids in the amino acid sequence represented by SEQ ID NO:2 may be, for example: (i) an amino acid sequence having 1-20 (preferably 1-10, more preferably 1-5 and still more preferably 1-2) amino acids deleted from the amino acid sequence represented by SEQ ID NO:2; (ii) an amino acid sequence having 1-20 (preferably 1-10, more preferably 1-5 and still more preferably 1-2) amino acids substituted with other amino acids in the amino acid sequence represented by SEQ ID NO:2; (iii) an amino acid sequence having 1-20 (preferably 1-10, more preferably 1-5 and still more preferably 1-2) amino acids added to the amino acid sequence represented by SEQ ID NO:2; (iv) an amino acid sequence having 1-20 (preferably 1-10, more preferably 1-5 and more preferably 1-2) amino acids inserted into the amino acid sequence represented by SEQ ID NO:2; and (v) an amino acid sequence having any combination of (i)-(iv) above.

The protein of the present invention also comprises a protein coded by a gene of the present invention that will be described later.

The protein of the present invention may further contain a peptide sequence for purification and/or a secretory signal peptide. As the peptide sequence for purification, a peptide sequence that is used in the art can be used. Examples of the peptide sequence for purification include a histidine tag sequence having an amino acid sequence with four or more, preferably six or more consecutive histidine residues, a T7 tag sequence, an amino acid sequence of a binding domain for glutathione of glutathione S-transferase, and an amino acid sequence of Protein A. A secretory signal peptide refers to a peptide region that plays a role of allowing cell membrane permeability of a protein bound to this secretory signal peptide. An amino acid sequence of such a secretory signal peptide and a nucleotide sequence coding therefor are well known in the art and have been reported (see, for example, von Heijine G (1988) Biochim. Biohys. Acra 947: 307-333, von Heijine G (1990) J. Membr. Biol. 115: 195-201, etc.). More specifically, examples of the secretory signal peptide include a secretory signal peptide derived from outer membrane protein A of *E.coli* (ompA) (Ghrayeb, J. et al. (1984) EMBO J. 3:2437-2442), and a secretory signal peptide derived from cholera toxin of *vibrio cholera*.

The protein of the present invention may further contain a peptide sequence for cleavage. A peptide sequence for cleavage is a sequence for excising a peptide sequence for purification and/or a secretory signal peptide from the protein of interest, an example being a thrombin-recognition sequence.

An amino acid sequence of a protein of one embodiment of the present invention is represented by SEQ ID NO:4. This protein is a protein having a thrombin recognition site, a T7 tag and a histidine tag fused in this order behind the amino acid sequence represented by SEQ ID NO:2.

A method for acquiring a protein of the present invention is not particularly limited. A protein of the present invention may be a chemically synthesized polypeptide or a recombinant protein produced by a genetic recombinant technique. In the case where a protein of the present invention is chemically synthesized, it may be synthesized, for example, by Fmoc method (fluorenylmethyloxycarbonyl method), tBoc method (t-butyloxycarbonyl method) or the like. Moreover, a peptide synthesizer provided by Advanced Chemtech, Perkin-Elmer, Pharmacia, Protein Technology Instrument, Synthecell-Vega, PerSeptive, Shimadzu or the like may be employed for chemical synthesis. In the case where a protein of the present invention is produced by a genetic recombinant technique, it may be produced by a general genetic recombinant technique. More specifically, a gene coding for the protein of the present invention can be introduced into an appropriate expression system to produce the protein of the present invention. Expression of a gene coding for the protein of the present invention in an expression system will be described later.

### (3) Gene of the invention

A gene of the present invention is a gene coding for a protein of the present invention. The protein of the present invention is as already described above. An example of the gene of the present invention includes, but not limited to, DNA consisting of the nucleotide sequence represented by SEQ ID NO:1.

The gene of the present invention include DNA consisting of a nucleotide sequence having homology (identity) of about 60% or more, preferably about 70% or more, more preferably about 80% or more, still more preferably about 90% or more, particularly preferably about 95% or more, yet particularly preferably about 98% or more with the nucleotide sequence represented by SEQ ID NO:1 and coding for a protein having a flowering-inducing activity.

The value of the above-mentioned identity is generally the larger the better. The identity of an amino acid sequence may be determined by using an analysis program such as BLAST (see, for example, Altzshul S. F. et al., J. Mol. Biol. 215, 403 (1990)). When using BLAST, the default parameters for each program are used.

Furthermore, the gene of the present invention comprises, other than the above-mentioned DNA, DNA consisting of a nucleotide sequence that has mutation of deletion, substitution and/or addition of one or several nucleotides in the nucleotide sequence represented by SEQ ID NO:1 and that codes for a protein having a flowering-inducing activity.

Moreover, the gene of the present invention also comprises DNA that hybridizes with DNA consisting of a nucleotide sequence complementary to the nucleotide sequence represented by SEQ ID NO:1 under stringent conditions and that codes for a protein having a flowering-inducing activity. The gene of the present invention comprises such genes.

An example of stringent conditions includes, but not limited to, conditions under which hybridization is performed while keeping a DNA-fixed nylon membrane warm together with a probe at 65°C for 20 hours in a solution containing 6 x SSC (1 x SSC is 8.76g of sodium chloride and 4.41g of sodium citrate dissolved in a liter of water), 1% SDS, 100 µg/ml salmon sperm DNA, 0.1% bovine serum albumin, 0.1% polyvinylpyrrolidone and 0.1% ficoll. Conditions of hybridization can appropriately be determined by those skilled in the art while considering, in addition to conditions such as salt concentration of the buffer and temperature, other conditions such as probe concentration, probe length and reaction time.

For detailed procedure of the hybridization method, see Molecular Cloning, A Laboratory Manual 2nd ed. (Cold Spring Harbor Laboratory Press (1989)) and the like.

Hereinafter, an example of a method for obtaining a gene of the present invention by hybridization will be described, although the present invention is not limited thereto.

First, DNA obtained from an appropriate gene source is linked to a plasmid or a phage vector according to a routine method to prepare a DNA library. A transformant obtained by introducing this library into a suitable host is cultured on a plate. The grown colony or plaque is transferred onto a nitrocellulose or nylon membrane, which is subjected to denaturation followed by fixation of DNA on the membrane. This membrane is kept warm under the above-described stringent conditions for hybridization in a solution having the above-described composition containing a probe labeled with ³²P or the like. The probe used may be any probe as long as it contains a polynucleotide coding for the entire or a part of the amino acid sequence represented by SEQ ID NO:2. For example, a polynucleotide consisting of or containing whole or a part of the nucleotide sequence represented by SEQ ID NO:1 can be used.

At the end of the hybridization, non-specifically adsorbed probe is washed away, and a clone that formed a hybrid with the probe is identified by autoradiography or the like. This process is repeated until a hybrid-forming clone is isolated.

The resulting clones retain a gene coding for a protein of interest having the flowering-inducing activity. In order to obtain a gene of interest from the clones, a known polynucleotide-extracting method such as an alkali-SDS method is used.

Furthermore, examples of a nucleotide sequence having mutation of deletion, substitution and/or addition of one or several nucleotides in the nucleotide sequence represented by SEQ ID NO:1 include: (i) a nucleotide sequence having 1-20 (preferably 1-10, more preferably 1-5 and still more preferably 1-2) nucleotides deleted from the nucleotide sequence represented by SEQ ID NO:1; (ii) a nucleotide sequence having 1-20 (preferably 1-10, more preferably 1-5 and still more preferably 1-2) nucleotides substituted with other nucleotides in the nucleotide sequence represented by SEQ ID NO:1; (iii) a nucleotide sequence having 1-20 (preferably 1-10, more preferably 1-5 and still more preferably 1-2) nucleotides added to the nucleotide sequence represented by SEQ ID NO:1; (iv) a nucleotide sequence having 1-20 (preferably 1-10, more preferably 1-5 and still more preferably 1-2) nucleotides inserted into the nucleotide sequence represented by SEQ ID NO:1; and (v) a nucleotide sequence having any combination of (i)-(iv) above.

Such various DNAs are comprised in the gene of the present invention for the following reasons. Specifically, a codon (a combination of three nucleotides) that designates an amino acid on a gene is known to exist in one to six types for a single type of amino acid. Therefore, a plurality of genes may exist for coding a certain amino acid sequence.

Genes are not always stably present in nature, and occurrence of mutation in that nucleotide sequence is not uncommon. Depending on the mutation in the gene, that mutation may not change the amino acid sequence coded (which is referred to as silent mutation), in which case, there are different genes coding for the same amino acid sequence. Accordingly, even when a gene coding for a certain amino acid sequence is isolated, the possibility that multiple types of genes coding for the same amino acid sequence may emerge during the passage of the organism containing that gene cannot be ruled out.

Moreover, to artificially prepare multiple types of genes coding for the same amino acid sequence is not difficult by using various genetic engineering technique.

For example, upon producing a genetically engineered protein, when the codon originally used in the gene coding for the protein of interest is not frequently used in the host, the expression level of the protein may be low. In this case, higher expression of the protein of interest can be intended by artificially converting the codon into a codon frequently used in the host without changing the amino acid sequence coded.

Thus, multiple types of genes coding for a certain amino acid sequence can not only artificially be produced, but may also be generated in nature. Therefore, even if a gene is not identical with the gene having the nucleotide sequence disclosed by the present invention, it can be comprised by the gene of the present invention as long as it is DNA coding for a protein that shows a flowering-inducing activity.

In addition, introduction of mutation into a gene can introduce mutation such as deletion, substitution and/or addition of one or several amino acids. Mutation can be introduced into an amino acid sequence or a gene according to a known procedure such as Kunkel method or Gapped duplex method with a mutation-introducing kit utilizing site-directed mutagenesis, for example, QuikChange^{™} Site-Directed Mutagenesis Kit (Stratagene), GeneTailor^{™} Site-Directed Mutagenesis System (Invitrogen), TaKaRa Site-Directed Mutagenesis System (Mutan-K, Mutan-Super Express Km, etc.: Takara Bio) or the like.

A nucleotide sequence of DNA can be confirmed by a common sequencing method. For example, it may be carried out by dideoxynucleotide chain termination method (Sanger et al. (1977) Proc. Natl. Acad. Sci. USA 74: 5463) or the like. A sequence may also be analyzed using a suitable DNA sequencer.

For a transformant prepared with a plasmid vector, the nucleotide sequence is determined by culturing it, for example, in a test tube if the host *E.coli* (*Escherichia coli*), or the like to prepare a plasmid according a routine method. The resulting plasmid may be used directly as a template or the insert fragment may be removed and subcloned into M13 phage vector or the like to determine the nucleotide sequence by a dideoxy method or the like. In the case of a transformant prepared with a phage vector, the nucleotide sequence can be determined essentially by the same operation. Basic experimental techniques for the above-described culture to nucleotide sequence determination are described, for example, in T. Maniatis et al. Molecular Cloning, A Laboratory Manual or the like. Alternatively, a sequence can automatically be determined with a commercially available sequencer (Applied Biosystems 3130 Genetic analyzer (Life Technologies), ABI PRISM 3100 DNA analyzer (Life Technologies), etc.).

Whether or not the resulting gene codes for the protein of interest can be confirmed by comparing the determined nucleotide sequence with the nucleotide sequence represented by SEQ ID NO:1. Alternatively, an amino acid sequence deduced from the determined nucleotide sequence can be compared with the amino acid sequence represented by SEQ ID NO:2.

The gene of the present invention may contain a polynucleotide (e.g., DNA) coding for a peptide sequence for purification and/or a polynucleotide (e.g., DNA) coding for a secretory signal peptide. As the polynucleotide coding for a peptide sequence for purification, a polynucleotide containing a nucleotide sequence coding for a peptide sequence for purification used in the art can be used. Examples of the peptide sequence for purification include those mentioned above. As a polynucleotide coding for a secretory signal peptide, a polynucleotide containing a nucleotide sequence coding for a secretory signal peptide known in the art can be used. Examples of the secretory signal peptide include those mentioned above.

The gene of the present invention may further contain a polynucleotide (e.g., DNA) coding for a peptide sequence for cleavage. As the polynucleotide coding for a peptide sequence for cleavage, a polynucleotide containing a nucleotide sequence coding for a peptide sequence for cleavage used in the art may be used. Examples of the peptide sequence for cleavage include those mentioned above.

A nucleotide sequence of DNA contained in a gene of one embodiment of the present invention is represented by SEQ ID NO:3. This DNA has DNA coding for a thrombin recognition site, DNA coding for a T7 tag and DNA coding for a histidine tag fused in this order behind DNA consisting of the nucleotide sequence represented by SEQ ID NO:1.

### (4) Method for producing protein of the invention

In order to produce a protein of the present invention, first, a recombinant vector is prepared by integrating a gene coding for the protein of the present invention. Then, a transformant produced by transformation with this recombinant vector is cultured to collect a protein having a flowering-inducing activity from the culture, thereby producing the protein of the present invention.

A recombinant vector can be prepared by inserting a transcription promoter upstream of, or in some cases a terminator downstream from, a gene coding for a protein of the present invention to construct an expression cassette, which is inserted into an expression vector. Alternatively, when a transcription promoter and/or a terminator is already present in the expression vector, the promoter and/or terminator in the vector can be utilized to insert a gene coding for this protein therebetween without the need of constructing an expression cassette. Here, a terminator may not always be necessary.

Herein, examples of the promoter include, but not limited to, a trc promoter, a lac promoter and a T7 promoter.

Herein, examples of the terminator include, but not limited to, a trp operon terminator and a T7 terminator.

In order to insert a gene of the present invention into a vector, a method that uses a restriction enzyme, a method that uses topoisomerase, a method that uses homologous recombination or the like can be employed. If necessary for insertion, an appropriate linker can be added. In addition, as a nucleotide sequence critical for translation into amino acid, a ribosome-binding sequence such as SD sequence or Kozak sequence is known, which may be inserted upstream to the gene. Upon insertion, a part of the amino acid sequence coded by the gene may be substituted.

The vector is not particularly limited as long as it retains the gene of the present invention, and a vector suitable for each host can be used. Examples of the vector include plasmid DNA, bacteriophage DNA, retrotransposon DNA and artificial chromosome DNA. For example, when *E.coli* is used as a host, examples of a vector include pET-28 b (+) (Merck), pTrc99A (GE Healthcare Bioscience), pACYC184 (Nippon Gene), pMW118 (Nippon Gene) and the like. Furthermore, modified versions of these vectors may also be used as necessary.

A transformant can be prepared by integrating the recombinant vector into a host.

The host is not particularly limited as long as it is capable of expressing a protein of interest after integration of the above-described recombinant vector. Examples of the host include bacteria such as *E.coli* (*Escherichia coli*), *Bacillus subtilis, Rhodococcus* and *Actinomyces,* yeasts (*Saccharomyces cerevisiae*), fungi, animal cells, plant cells and insect cells. The host is preferably *E.coli.*

Examples of *E.coli* include *E.coli* K12 and B strains as well as strains derived from wild-type strains of these strains, namely, JM109 strain, XL1-Blue strain (e.g., XL1-Blue MRF'), K802 strain, C600 strain and Origami B(DE3) strain.

A technique for introducing a recombinant vector into a host is not particularly limited as long as it is suitable for the host, which may appropriately be selected from known techniques by those skilled in the art. Examples of such method include electroporation techniques, techniques utilizing calcium ion, spheroplast techniques, lithium acetate techniques, calcium phosphate techniques and lipofection techniques.

A protein of the present invention can be produced by culturing the transformant, and collecting a protein having a flowering-inducing activity from the resulting culture.

A "culture" refers to those that can be obtained by culturing, for example, a bacteria cell, a culture solution, a cell-free extract, a cell membrane or the like. A cell-free extract can be obtained, for example, by physically rupturing the cultured bacteria cell with a homogenizer or the like after addition of, for example, a sodium phosphate buffer, centrifuging the resultant and collecting the supernatant so that unruptured bacteria cell (cell) no longer exists. A cell membrane can be obtained as a pellet resulting from the above-described centrifugation, or obtained by suspending the pellet in a dissolving buffer.

A protein of the present invention can be purified from the culture employing a known method such as dialysis or ammonium sulfate precipitation or various chromatographies such as gel filtration, ion exchange and affinity, alone or in an appropriate combination. In this case, concentration and purification can be carried out by using a molecular weight, isoelectric point as an index (e.g., SDS-PAGE).

Depending on an expression system used, the protein of the present invention expressed in the transformant might be accumulated as an insoluble matter (inclusion body). In this case, this insoluble matter is collected, and solubilized under mild denaturation conditions, for example, in the presence of a denaturing agent such as urea, followed by removal of the denaturing agent, thereby obtaining an active-type protein. Furthermore, chromatography operation can be performed to purify a protein of interest having a flowering-inducing activity.

In addition, the above-obtained gene is used as a probe to perform hybridization under stringent conditions to obtain a gene coding for a protein having similar flowering-inducing activity whose sequence is slightly different from that of the resulting gene (for example, a gene containing DNA consisting of the nucleotide sequence represented by SEQ ID NO:1).

When the resulting gene does not contain the entire region coding for the protein of interest, primers are synthesized based on the nucleotide sequence of the resulting gene, so as to amplify the region lacking by PCR using the same, or the resulting gene fragment is used as a probe to repeat screening of the DNA library, thereby obtaining the entire coding region.

Thus, a transformant containing the gene of the present invention is prepared to express the protein of the present invention encoded by the gene. The expressed protein is purified. Preparation of the transformant, and expression and purification of the protein may all be carried out in the same manner as described above.

A protein of the present invention may also be produced from a gene of the present invention or an above-described vector. Specifically, a so-called cell-free protein synthesis system can be employed to produce a protein of the present invention.

A cell-free protein synthesis system is a system that synthesizes a protein using a cell extract in an artificial container such as a test tube. Here, a cell-free protein synthesis system also comprises a cell-free transcription system that synthesizes RNA by using DNA as a template.

Here, the above-described cell extract used may be an extract derived from an eukaryotic or prokaryotic cell, for example, an extract from wheat germ, *E.coli* or the like. These cell extracts may or may not be concentrated.

The cell extract may be obtained, for example, by ultrafiltration, dialysis, polyethyleneglycol (PEG) precipitation or the like. In addition, cell-free protein synthesis may also be carried out using a commercially available kit. Examples of such kit include reagent kit PROTEIOS^{™} (Toyobo) and TNT^{™} System (Promega) as well as synthesizers PG-Mate^{™} (Toyobo) and RTS (Roche Diagnostics).

The protein of the present invention obtained by cell-free protein synthesis described above may be concentrated and purified by appropriately selecting the above-mentioned chromatography.

### (5) Flowering inducer and method using flowering inducer

A flowering inducer contains a protein of the present invention as an active component. By administering the flowering inducer into a plant body, flowering of the plant body can be induced. Moreover, in order to produce the above-described flowering inducer, the protein of the present invention can be used.

A plant targeted for administration of the flowering inducer is not particularly limited as long as the plant can bear a flower, examples being fruit trees, trees such as pine, cedar and Japanese cypress, flowering trees (ornamental trees) such as azalea, camellia and sasanqua, vegetables such as carrot, cucumber, *daikon* radish, pumpkin, eggplant, tomato, cabbage, potato, Chinese cabbage, crown daisy, Japanese mustard spinach, sweet pepper, Welsh onion, onion, lettuce, ginger, garlic and strawberry, grains such as rice, wheat and corn, flowers such as chrysanthemum, tulip and rose, legumes such as soybean and pea, and the model plant *Arabidopsis.* Plants targeted for administration are preferably plants whose juvenile stage, the period from germination to flower setting, is relatively long (10 years or longer, 9 years or longer, 8 years or longer, 7 years or longer, 6 years or longer, 5 years or longer, 4 years or longer or 3 years or longer), for example, fruit trees. Since fruit trees are woody and the number of the plants cultivated is less compared to other agricultural plants, administration of the flowering inducer of the present invention can be relatively easy. Examples of fruit trees include citrus, kumquat, apple, Japanese plum, peach, grape, pear, loquat, persimmon and fig.

More preferably, the plant targeted for administration is citrus. Examples of citrus include mandarins (e.g., *Citrus unshiu, Citrus tangerina, Citrus tachibana, Citrus kinokuni,* King mandarin, clementine, Kara mandarin, etc.), oranges (e.g., Valencia orange, Navel orange, blood orange, hamlin orange, etc.), grapefruits (e.g., grapefruit, Sweetie, oroblanco, etc.), acid citruses (e.g., *yuzu,* bitter orange, *Citrus sphaerocarpa, Citrus sudachi,* lemon, *Citrus depressa,* lime, citron, *Citrus junos Tanaka Silbes, Citrus yuko,* etc.), natural hybrid citruses (e.g., *Citrus natsudaidai, Citrus natsudaidai Hayata f. Kawanonatsudaidai (Amanatsu), Citrus hassaku, Citrus tamurana, Citrus jabara, Citrus grandis, Citrus sulcata, Naruto* orange, *Citrus obovoidea,* etc.), tangors (*Citrus iyo, Citrus unshiu x sinensis, Harumi, Citrus tankan Hayata,* Murcott tangor, ortanique, etc.), tangelos (e.g., Seminole, Yalaha, Minneola, Orlando, etc.), pummelos (e.g., Kao Phuang pummelo, Banpeiyu pummelo, Egami-buntan pummelo, Hirado-buntan pummelo, Mato-buntan pummelo, etc.), trifoliate oranges (trifoliate orange, Flying Dragon, Rubidoux, etc.) and kumquats (Meiwa kumquat, round kumquat, oval kumquat, etc.). More preferably, the plant targeted for administration is *Citrus unshiu.*

In some preferable embodiments of the present invention, the plant targeted for administration is a plant obtained by grafting a scion in the mature stage to a rootstock. Examples of the scion include the above-mentioned plants targeted for administration of the flowering inducer, and it is preferably the above-mentioned fruit trees, more preferably the above-mentioned citrus and still more preferably *Citrus unshiu.* As the rootstock, a known rootstock can be used, examples including the above-mentioned plants targeted for administration of the flowering inducer, and it is preferably the above-mentioned fruit trees, more preferably the above-mentioned citrus and still more preferably trifoliate orange.

For example, the juvenile stage of a citrus hybrid seedling lasts for 8-12 years. A citrus grafted with a scion in the mature stage can be treated with a flowering inducer of a preferable embodiment so that the period before the flowering can be reduced to be within 2 years, thereby realizing earlier harvesting of the citrus fruit.

A flowering inducer contains the protein of the present invention as an active component. Other than the protein of the present invention, it may also contain auxiliary agents such as commonly used carrier, emulsifier, dispersant, spreading agent, wetting agent, fixing agent, disintegrant and the like.

Examples of carrier include water; alcohols such as ethanol, methanol, isopropanol, butanol, ethylene glycol and propylene glycol; ketones such as acetone, methylethylketone and cyclohexane; esters such as ethyl acetate; and solid carriers, for example, talc, bentonite, clay, kaolin, diatomite, white carbon, vermiculite, calcium hydroxide, silica sand, ammonium sulfate and urea.

As an emulsifier or a dispersant, a surfactant is usually used. Examples include anionic surfactants such as higher alcohol sodium sulfate, cationic surfactants such as stearyl trimethylammonium chloride, and non-ionic surfactants such as polyoxyethylene alkyl phenyl ether.

The dosage form of the flowering inducer is not particularly limited, and examples include an emulsion, a suspension, powder, a hydrating agent, a water-soluble agent, granules, paste and aerosol.

Although the time used and the method of usage of a flowering inducer may appropriately be selected according to the type of the plant, the flowering inducer is injected into a plant, for example, a plant obtained by grafting a scion in the mature stage into a rootstock. According to an "injection method", a dropper, absorbent cotton impregnated with a flowering inducer or the like may be used for injecting the inducer into a plant body from a stalk, a leaf, a root or the like of the plant body.

The content of the protein of the present invention in the flowering inducer and the dosage of the flowering inducer differ depending on the type of the plant, dosage form, method of usage, time to be used and the like. For example, when the flowering inducer is injected as a water-soluble agent into a citrus, the protein of the present invention is adjusted such that its concentration lies in a range of 20-10000 ppm, preferably 50-10000 ppm, more preferably 100-10000 ppm, and injected into the citrus at an amount of 0.1-5.0 ml, preferably 0.2-2.0 ml, and more preferably 0.5-1.0 ml during the physiological floral bud differentiation period of the citrus.

The present invention also provides a method for inducing flowering, which comprises, for example, injecting the protein of the present invention into a plant of interest, and placing a plant injected with the protein of the present invention next to a plant of interest. The phrase "placing next to" comprises arrangement such that the distance between the main stem (the main stem stays almost at the center of the plant body) of a plant administered with the protein of the present invention and the main stem of a plant arranged next to it is, for example, within 2 m, within 1.9 m, within 1.8 m, within 1.7 m, within 1.6 m, within 1.5 m, within 1.4 m, within 1.3 m, within 1.2 m, within 1.1 m, within 1 m, within 0.9 m or within 0.8 m. According to a method for inducing flowering of the present invention, a plant targeted for administration, an auxiliary agent, time to be used, method of usage, a content of the protein of the present invention, a dosage of a flowering inducer and the like are the same as those described for the above-described flowering inducer.

A flowering inducer or a method for inducing flowering according to a preferable embodiment has at least one effect selected from the group consisting of the followings: (a) the cost spent on cultivation until bearing can be reduced since the time of bearing can be made earlier; (b) investment upon opening a farm can be recovered earlier since the time of bearing can be made earlier; and (c) early flowering can result in efficient variety improvement.

A flowering inducer or a method for inducing flowering according to a more preferable embodiment can prevent alternate year bearing of a citrus, in particular *Citrus unshiu*.

Moreover, a flowering inducer or a method for inducing flowering according to some embodiments does not use a recombinant plant, as an alternative method for inducing flowering.

Here, the best modes and specific examples for carrying out the present invention are preferable embodiments of the present invention, which are provided for illustration or explanation and the present invention is not limited thereto.

### EXAMPLE 1

### 1. PCR of CiFT2 gene

Complementary DNA having high homology with complementary DNA of FT was obtained by RT-PCR method using RNA extracted from *Citrus unshiu* leaf as a template. Specifically, the method was carried out as follows. Primers used for RT-PCR method were designed based on cDNA sequence of *Citrus unshiu* CiFT (Accession No. AB027456, SEQ ID NO:5) and cDNA sequence of *Citrus unshiu* CiFT2 whose DNA sequence is 99% identical to that of CiFT (Accession No. AB301934, SEQ ID NO:7) (Fumie Nishikawa, Tomoko Endo, Takehiko Shimada, Hiroshi Fujii, Tokurou Shimizu, Mitsuo Omura, and Yoshinori Ikoma (2007) "Increased CiFT abundance in the stem correlates with floral induction by low temperature in Satsuma mandarin (Citrus unshiu Marc.)." J. Exp. Bot. 58, 3915-3927). Amino acid sequences deduced from the nucleotide sequences represented by SEQ ID NOS:5 and 7 are represented by SEQ ID NOS:6 and 8, respectively.

TRIZOL (Invitrogen) was used to extract RNA from *Citrus unshiu* leaf according to the attached instruction. Reverse transcription reaction was carried out with SuperScript II (Invitrogen) enzyme using the extracted RNA as a template and a primer having DNA sequence of CAAATTAAGCATGTATGG (SEQ ID NO:9) under the conditions described in Piero Carninci, Yoko Nishiyama, Arthur Westover, Masayoshi Itoh, Sumiharu Nagaoka, Nobuya Sasaki, Yasushi Okazaki, Masami Muramatsu, and Yoshihide Hayashizaki (1998) "Thermostabilization and thermoactivation of thermolabile enzymes by trehalose and its application for the synthesis of full length cDNA" Proc. Natl. Acad. Sci. USA. 95, 520-524, thereby obtaining complementary DNA.

Then, this complementary DNA was used as a template to perform nested PCR. Primers having the following DNA sequences were used in the first PCR reaction.
Primer: CAAATTAAGCATGTATGG (SEQ ID NO:10)
Primer: GTGCTTAGTGTTGTTGAG (SEQ ID NO:11)

Next, the PCR reaction was conducted using the above-mentioned primers as well as PrimeSTAR GXL DNA Polymerase (Takara Bio) according to the attached instruction. The reaction took place by repeating 20 cycles of: 98°C for 10 seconds; 55°C for 15 seconds; and 68°C for 1 minute.

Primers having the following DNA sequences were used in the second PCR reaction.
Primer: AGGAGATATACCATGTCTAGCAGGGAGAGAGA (SEQ ID NO:12)
Primer: CACCAGGCCGCTGCTTCGTCTGACAGGCCTTCCGC (SEQ ID NO:13)

Then, PCR reaction was performed using the above-mentioned primers as well as PrimeSTAR GXL DNA Polymerase according to the attached instruction. The reaction took place by repeating 20 cycles of: 98°C for 10 seconds; 55°C for 15 seconds; and 68°C for 1 minute.

Primers having the following DNA sequences were used in the third PCR reaction.
Primer: AATTTTGTTTAACTTTAAGAAGGAGATATACCATG (SEQ ID NO:14)
Primer: GCCATATGGCTGCCGCGCGGCACCAGGCCGCTGCT (SEQ ID NO:15)

Subsequently, PCR reaction was performed using the above-mentioned primers as well as PrimeSTAR GXL DNA Polymerase according to the attached instruction. The reaction took place by repeating 40 cycles of: 98°C for 10 seconds; 55°C for 15 seconds; and 68°C for 1 minute.

Here, the primer sequences used for the above-described second and third PCR reactions contain a sequence for adding a sequence homologous to plasmid pET-28b(+) (Merck) to a PCR product.

### 2. Cloning PCR product into plasmid pET-28b(+)

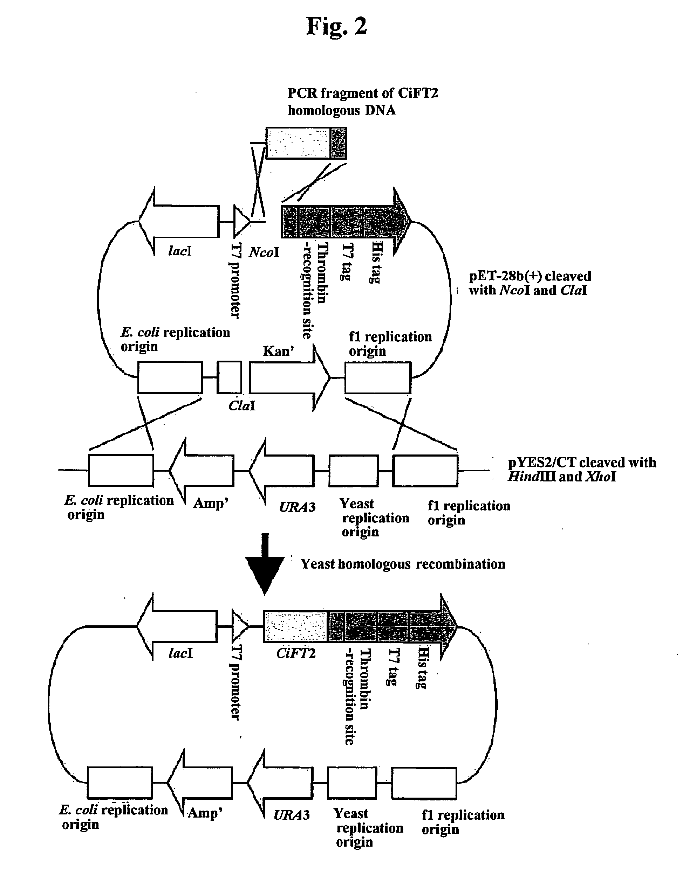

The PCR product was cloned into the plasmid according to an yeast homologous recombination technique (Ei'ichi Iizasa and Yukio Nagano (2006) "Highly efficient yeast-based in vivo DNA cloning of multiple DNA fragments and the simultaneous construction of yeast/ Escherichia coli shuttle vectors" BioTechniques 40, 79-83). As shown in Figure 2, plasmid pET-28b(+) cleaved with restriction enzymes NcoI and ClaI, plasmid pYES2/CT (Invitrogen) cleaved with restriction enzymes HindIII and XhoI, and the above-described PCR product were simultaneously transformed into an yeast. As a result, due to homologous recombination reaction within the yeast cell, pET-28b(+) will gain the replication origin and selective marker URA3 of the yeast while the PCR product is cloned into pET-28b(+). This experiment was conducted according to Ei'ichi Iizasa and Yukio Nagano (2006) BioTechniques 40, 79-83. The resulting plasmid allows expression of a protein having a thrombin recognition site, a T7 tag as an epitope tag, and a His tag for purification fused in this order behind FT protein. A population of the transformed yeast colonies was collected, from which plasmid DNA was purified. Subsequently, the purified plasmid DNA was transformed into *E.coli.* Plasmid DNA was purified from one of the resulting *E.coli* colonies to determine the DNA sequence having the PCR product inserted.

The resulting DNA sequence is represented by SEQ ID NO:1. In addition, an amino acid sequence deduced from this DNA sequence is represented by SEQ ID NO:2. This DNA sequence was the same as that of *Citrus unshiu* CiFT2. This CiFT2 codes for CiFT2 protein.

### 3. Comparison of amino acid sequence of CiFT2 protein

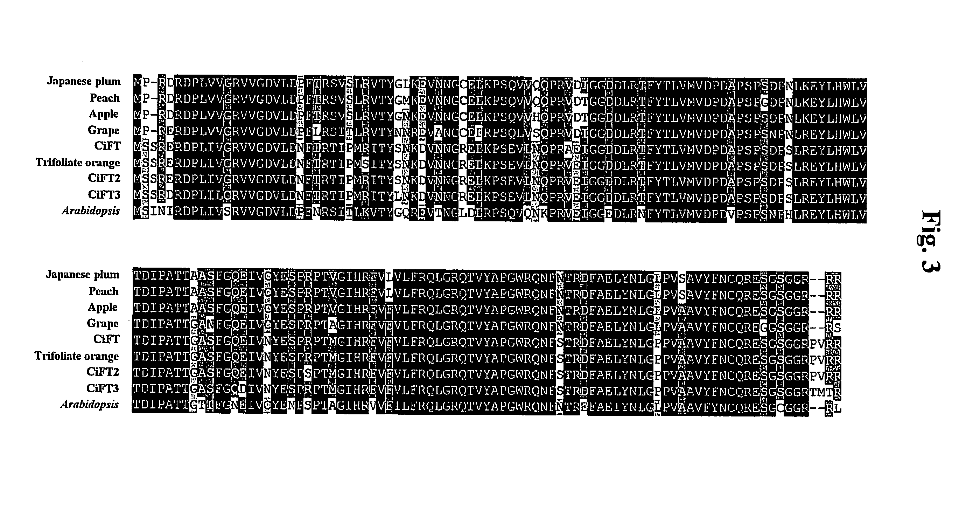

Figure 3 show the results from comparing with amino acid sequences of FT proteins from some plants. CiFT2 protein shows 99% and 95% identity with *Citrus unshiu*-derived CiFT protein and *Citrus unshiu*-derived CiFT3 protein, respectively. Moreover, CiFT2 protein also shows 99%, 84%, 83%, 82% and 81% identity with the amino acid sequences of trifoliate orange-, apple-, Japanese plum-, peach- and grape-derived FT proteins, respectively. Meanwhile, CiFT2 protein shows 75% identity with the amino acid sequence of *Arabidopsis-*derived FT protein. Accordingly, the amino acid sequence of CiFT2 protein is relatively similar with fruit tree-derived FT proteins than with *Arabidopsis*-derived FT proteins.

### 4. Production of CiFT2 protein with E.coli transformant

Next, CiFT2 protein was produced with *E. coli.* The constructed plasmid was transformed into *E. coli* Origami B (DE3) (Merck). This *E. coli* was cultured in 250ml of Terrific medium (24 g of yeast extract, 12 g of peptone, 9.4 g of dipotassium hydrogen phosphate, 2.2 g of potassium dihydrogen phosphate and 4 ml of glycerol per liter of water) containing 100 µg/ml ampicillin under the conditions of 37°C until absorbance at 600 nm became about 0.7. Then, IPTG was added to 1 mM and cultured at 15°C for two days.

The cultured *E.coli* was harvested and suspended in 40 ml of phosphate buffered saline (a buffer obtained by dissolving 8 g of sodium chloride, 0.2 g of potassium chloride, 1.44 g of disodium hydrogen phosphate and 0.24 g of potassium dihydrogen phosphate in 800 ml of water, whose pH was adjusted to 7.4 and then made 1 liter with water). To this suspension, phenylmethylsulfonyl fluoride (inhibitor of proteinase), deoxyribonuclease (DNA hydrolase) and lysozyme (enzyme that lyses bacterial cell walls) were added to 1 mM, 10 µg/ml and 100 µg/ml, respectively, for reaction at 30°C for 15 minutes.

Subsequently, a French press was used to disrupt this reaction solution under the pressure conditions of about 150 MPa. In order to remove the insoluble fraction from the cell fracture solution, centrifugation was performed at 20,000g for 10 minutes twice. The supernatant was passed through 10 ml of Chelating Sepharose Fast Flow (GE Healthcare Bioscience) charged with nickel ion. Then, the resultant was washed with 100 ml of wash buffer 1 (50 mM phosphate buffer (pH 7.0), 0.5 M sodium chloride, 1% Triton X-100), followed by 20 ml of wash buffer 2 (50 mM phosphate buffer (pH 7.0), 0.5 M sodium chloride) and finally with 20 ml of wash buffer 3 (50 mM phosphate buffer (pH 7.0), 0.5 M sodium chloride, 100 mM imidazole).

Next, the recombinant protein was eluted for a total of six times with 5 ml of elution buffer (50 mM phosphate buffer (pH 7.0), 0.5 M sodium chloride, 200 mM imidazole) (total 30 ml). Since larger amounts of the recombinant protein were contained in the third to sixth elution solutions, these fractions were subjected to dialysis to remove imidazole. The dialysis took place for a total of five times with 25-fold phosphate buffered saline. The concentration of the resulting protein was determined with Protein Assay from BIO-RAD (Bradford assay) according to the attached instruction.

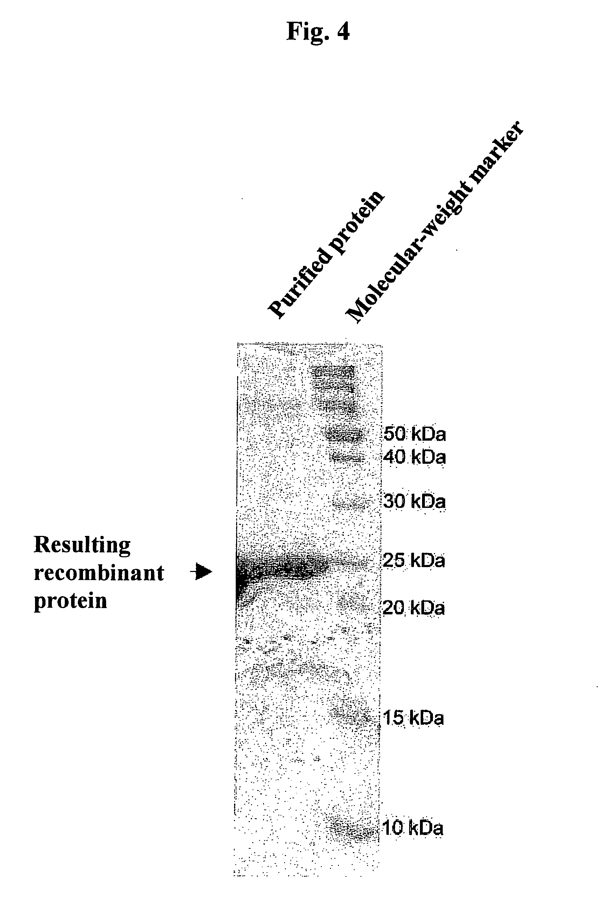

Figure 4 shows Coomassie Brilliant Blue staining after electrophoresing a part of the purified proteins with 14% SDS polyacrylamide gel. It shows that purification was relatively high. Relative molecular weight of the recombinant protein was almost the same as the predicted value. Here, a thrombin recognition site, a T7 tag as an epitope tag and a histidine tag for purification are fused in this order behind the CiFT2 protein, but these extra sequences were left and used in the subsequent examples.

A nucleotide sequence of DNA coding for the obtained recombinant protein is represented by SEQ ID NO:3. Moreover, an amino acid sequence deduced from the nucleotide sequence is represented by SEQ ID NO:4.

### 5. Administration of flowering inducer and results thereof (No. 1)

### 5. 1. Administration of flowering inducer

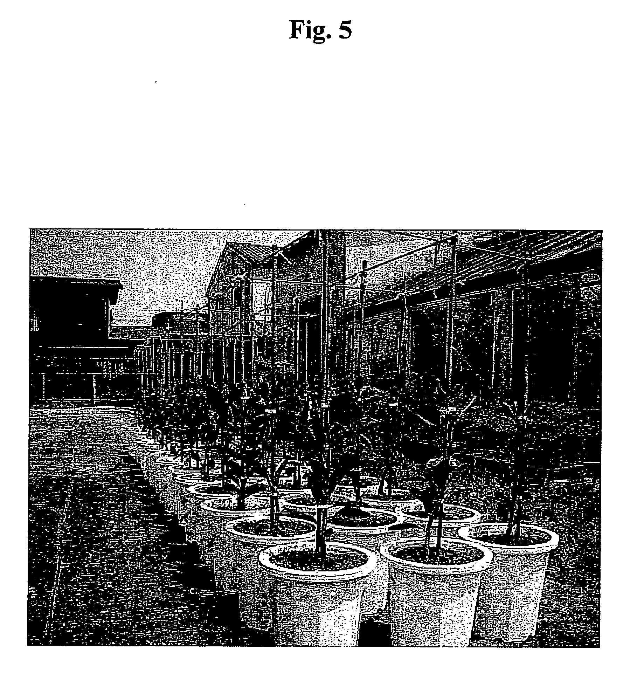

Then, a flowering inducer containing the above-obtained recombinant CiFT2 protein was injected into *Citrus unshiu.* The plant used was a one-year-old seedling, which can be obtained 12 months (April, 2008) after grafting *Citrus unshiu* (variety: Aoshima unshiu) as a scion to trifoliate orange as a rootstock. Injection of the flowering inducer into *Citrus unshiu* was carried out according to the following two methods. For reference, a picture of a one-year-old seedling taken in April, 2009 is shown in Figure 5.

### Injection method 1)

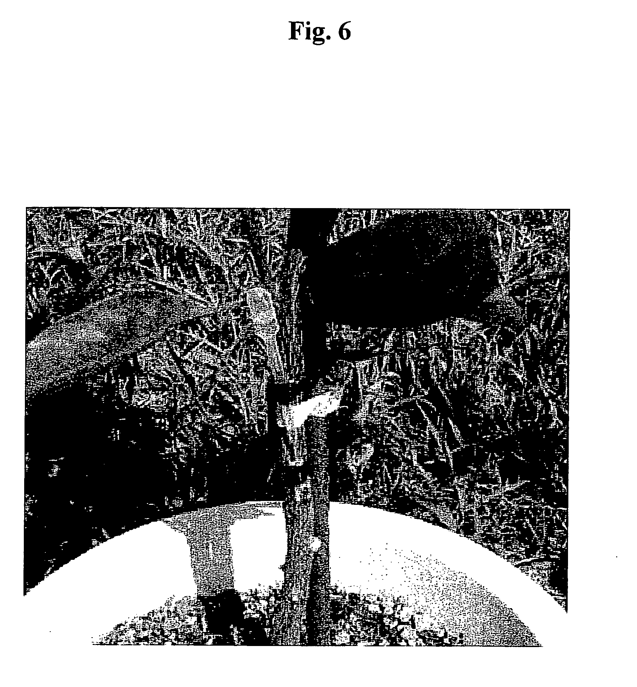

A flowering inducer was injected in September, October and November, 2008. Injection was performed on the upper part of the grafted site on the main stem. The above-obtained recombinant CiFT2 protein was diluted in a phosphate buffered saline to prepare a 500 ppm flowering inducer. One ml of 500 ppm (0.5 mg/ml) flowering inducer was drawn into a plastic dropper. Then, the epidermis was cut to the xylem, into which the tip of the plastic dropper was inserted where the inserted region was covered by an adhesive (Aron Alpha^{™} (Toagosei)) so as to prevent leaking of the liquid of the flowering inducer (Figure 6). The dropper was secured to the stalk using a tape. Note that the liquid was not injected by pressing the dropper but by natural absorption.

Note that during this time of year (September, October and November), *Citrus unshiu* is in the physiological floral bud differentiation period. As described above, while a substance for inducing flowering is synthesized in the leaves, this flowering-inducing substance is found to be synthesized during the physiological floral bud differentiation period based on the experiments in which leaves were removed from a plant body at various period. There is no change in the morphology that can be observed with a microscope during the physiological floral bud differentiation period. Actual flowering takes place in next April.

### Injection method 2)

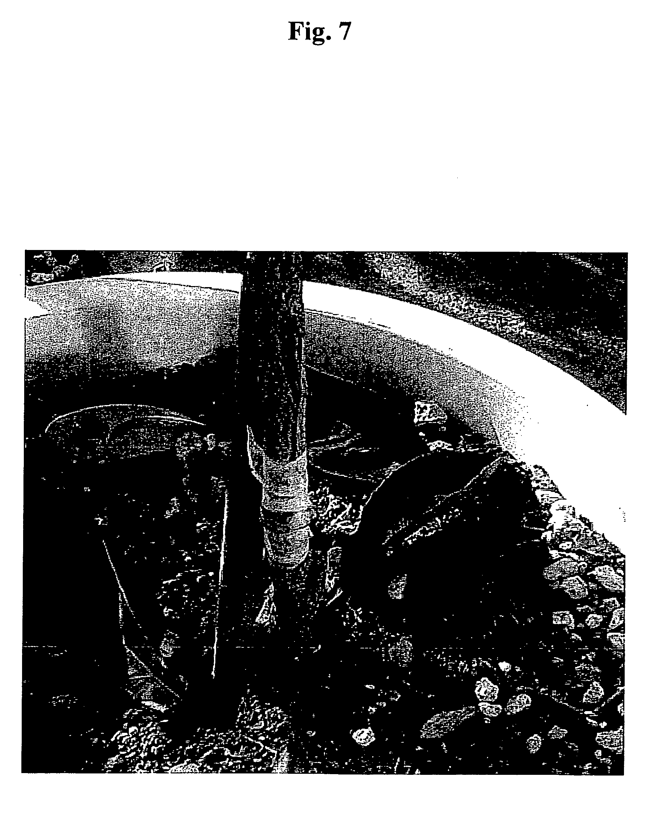

The flowering inducer was injected into the upper part of the grafted site on the main stem in April, 2008. After cutting into the epidermis to the xylem with knife, an absorbent cotton impregnated with about 0.2 ml of the flowering inducer was placed on the cut and wrapped with a tape (tape used for grafting) to inject the flowering inducer (Figure 7). The concentration of the flowering inducer impregnated by the absorbent cotton was 10,000ppm, 1,000ppm or 100ppm. The flowering inducers at these concentrations were prepared by diluting the above-obtained recombinant CiFT2 protein in a phosphate buffered saline.

### 5. 2. Results from flowering inducer administration

Apparent formation of buds was observed in most of the treated plants at the beginning of April, 2009. They flowered from the end of April to the beginning of May.

The following Table 1 shows the results of the experiment carried out according to Injection method 1. In this table, a vegetative shoot (non-flowering shoot) literally refers to a shoot that develops without flower setting (vegetative growth). There are two types of floral buds, namely, leafy flower associated with leaves and leafless flower, i.e., a floral bud with flower only. In general, about 90% of the leafless flower is associated with flower abscission or fruit abscission and thus even if it bears a fruit, it is often poor in quality. On the other hand, a leafy flower has a higher bearing rate, and its fruit is often good in quality.

As can be appreciated from Table 1, a significant number of flower setting occurred to some level in most of the plants, and the number of leafy flowers was larger than that of leafless flowers. The results obtained with the control plant (untreated plant) will be described later, but in general, a seedling of Aoshima unshiu, one variety of *Citrus unshiu,* is usually still at the middle of vegetative growth after a year of plant settling and rarely bears a flower. There was no apparent difference among the months of treatment. The reason for less flower setting in No. 4 that was treated in October is unclear.

**[Table 1]**

| No. | Month of treatment | Vegetative shoot (Non-flowering shoot) | Leafy flower (Flowering shoot) | Leafless flower |
|---|---|---|---|---|
| 1 | September | 46 | 65 | 6 |
| 2 | September | 9 | 52 | 10 |
| 3 | September | 4 | 108 | 66 |
| 4 | October | 48 | 4 | 3 |
| 5 | October | 0 | 91 | 55 |
| 6 | November | 18 | 56 | 22 |
| 7 | November | 51 | 57 | 0 |
| 8 | November | 39 | 31 | 2 |

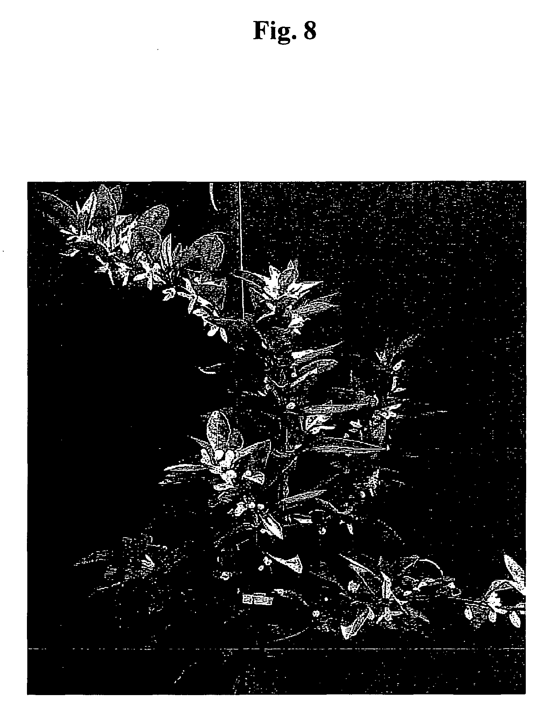

The picture of plant No.3 in Table 1 above is shown in Figure 8. In Figure 8, a number of white leafy flowers are setting.

The results from the experiment according to injection method 2 are shown in Table 2 below. A significant number of flower setting occurred to some level in most of the plants, and the number of leafy flowers was larger than that of leafless flowers. There was no apparent difference among the concentrations of treatment.

**[Table 2]**

| No. | Concentration (ppm) | vegetative shoot (non-flowering shoot) | leafy flower (flowering shoot) | leafless flower |
|---|---|---|---|---|
| 9 | 10,000 | 8 | 56 | 24 |
| 10 | 10,000 | 29 | 29 | 4 |
| 11 | 10,000 | 7 | 119 | 100 |
| 12 | 10,000 | 27 | 46 | 11 |
| 13 | 10,000 | 1 | 82 | 74 |
| 14 | 10,000 | 0 | 39 | 83 |
| 15 | 1,000 | 55 | 17 | 0 |
| 16 | 1,000 | 29 | 36 | 0 |
| 17 | 1,000 | 16 | 91 | 33 |
| 18 | 100 | 26 | 74 | 7 |
| 19 | 100 | 27 | 51 | 2 |
| 20 | 100 | 12 | 75 | 6 |
| 21 | 100 | 3 | 89 | 101 |
| 22 | 100 | 23 | 42 | 2 |
| 23 | 100 | 11 | 32 | 6 |

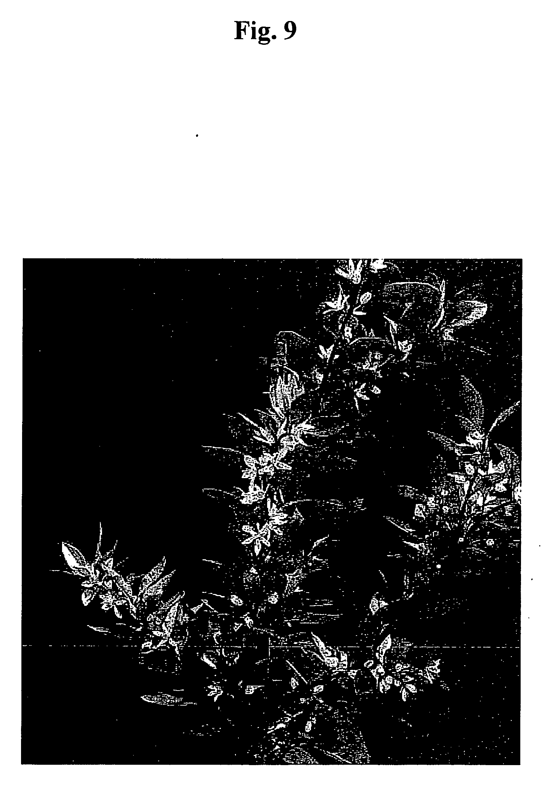

The picture of plant No.21 in Table 2 above is shown in Figure 9. In Figure 9, a number of white leafy flower are setting.

Results obtained in a land section adjacent (within 80 cm) to the plants treated according to Injection method 2 are shown in Table 3 below. Similar to Table 2, a significant number of flower setting occurred to some level in most of the plants, and the number of leafy flowers was larger than that of leafless flowers. The reason why flower setting occurred in this land section, i.e., untreated control land section, will be argued later.

Here, the distance to the land section adjacent to the plant treated according to Injection method 2 refers to a distance from the main stem of the treated plant to the main stem of the plant arranged in the adjacent land section.

**[Table 3]**

| No. | Vegetative shoot (Non-flowering shoot) | Leafy flower (Flowering shoot) | Leafless flower |
|---|---|---|---|
| 24 | 16 | 73 | 35 |
| 25 | 10 | 70 | 7 |
| 26 | 15 | 64 | 16 |
| 27 | 7 | 78 | 45 |
| 28 | 2 | 84 | 37 |
| 29 | 2 | 115 | 12 |

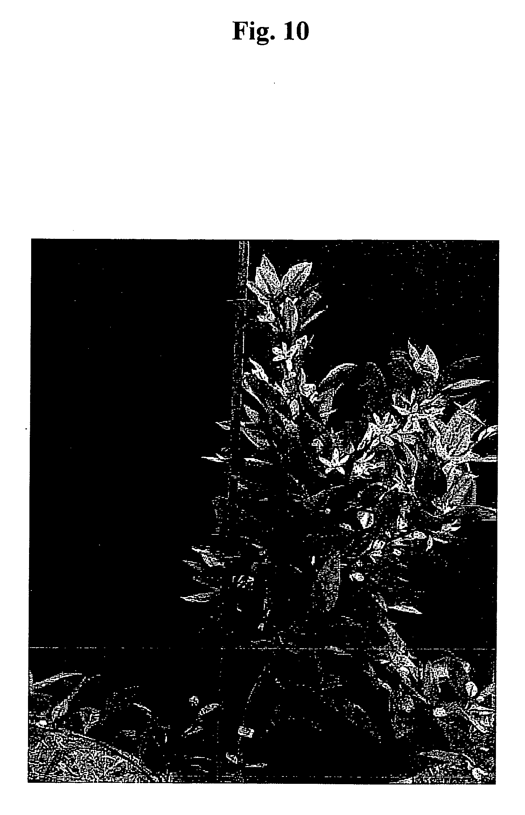

A picture of the plant No.24 in Table 3 is shown in Figure 10. Figure 10 shows a number of white leafy flowers setting.

Results from experiment on a land section that was farther than the land section adjacent (within 80 cm) to the plant treated according to injection method 2 are shown in Table 4 below. Here, the "distance" refers to a distance from the land section adjacent to the plant treated according to Injection method 2. In Table 4, distances from the land section adjacent to the plant treated according to Injection method 2 are shown. The number of flower setting tended to decrease depending on the distance. As mentioned earlier, in general, a seedling of Aoshima unshiu, one variety of *Citrus unshiu,* is usually still at the middle of vegetative growth after a year of plant settling and rarely bears a flower. In particular, this tendency was significant for outdoor cultivation where plants are directly planted in a farm field than planting in plant pots like in the above cases. Accordingly, the state of No. 37 is likely to be the most usual state.

**[Table 4]**

| No. | Distance (m) | Vegetative shoot (Non-flowering shoot) | Leafy flower (Flowering shoot) | Leafless flower |
|---|---|---|---|---|
| 30 | 1 | 43 | 30 | 5 |
| 31 | 1 | 44 | 20 | 0 |
| 32 | 1.6 | 1 | 42 | 28 |
| 33 | 1.9 | 22 | 55 | 3 |
| 34 | 2.6 | 52 | 14 | 0 |
| 35 | 2.9 | 27 | 20 | 4 |
| 36 | 2.9 | 40 | 26 | 10 |
| 37 | 2.9 | 55 | 0 | 0 |

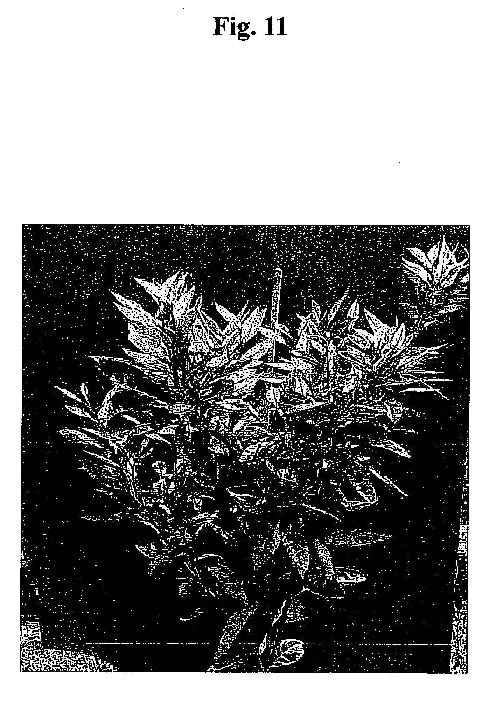

Figure 11 is a picture of a plant that was placed 2.9 m away. Some parts of the plant body (for example, the surface of the leaves) look white from the light reflection but no flower setting can be observed.

At the same time as flowering, other phenomenon was also observed. *Citrus unshiu* inherently has male sterility and may develop pollen by warming in the greenhouse cultivation, but rarely develops pollen by outdoor cultivation. From this series of experiments, however, it was found that many of the flowers borne in the present experiment developed pollen through dehiscence of the anther wall. The valuable *Citrus unshiu* pollen can be used for cross breeding. Due to this pollen development, a citrus having male sterility and thus has been impossible to be used as a pollen parent may be used as a pollen parent. This may lead to efficiency in variety improvement of the citrus.

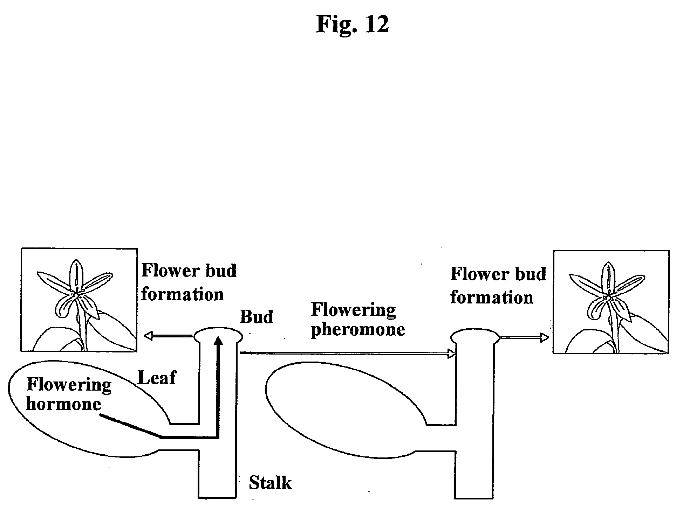

Now, in this series of experiments, flower setting was also observed for a tree that was not treated with the flowering inducer, but placed next to a tree treated with the flowering inducer. In addition, the number of flower setting of the tree not treated with the flowering inducer became less as it got farther from the tree treated with the flowering inducer. Usually, a seedling of Aoshima unshiu, a variety of *Citrus unshiu,* rarely bears a flower after a year of plant settling. One possibility of the mechanism of flower setting of a plant not treated with the flowering inducer but placed next to the plant injected with the flowering inducer is that an unknown flowering-inducing substance which may be called a "flowering pheromone" may possibly be released from the plant injected with the flowering inducer (Figure 12). Hereinafter, this hypothesis will be explained, but the present invention is not limited to the case where flowering induction arises due to the mechanism of the following hypothesis.

A pheromone is a physiologically active substance generated in an individual and secreted outside the individual to trigger a change in a certain behavior or development of another individual of the same species. Communication among individuals via a pheromone is a widely found phenomenon in nature. A phenomenon found by the present inventors can be explained by this communication among individuals via a pheromone.

In many fruit trees including mandarin orange, a phenomenon called alternate year fruitage ("fruitage" herein means to "bear a fruit") is known to occur, which is a phenomenon that repeats rich and poor harvests every other year in the entire fruit orchard. The reason for this alternate year fruitage, however, is unclear. Alternate year fruitage is a phenomenon where flower setting are synchronous in the entire fruit orchard. This phenomenon called alternate year fruitage may possibly be explained by communication among individuals via a pheromone.

Furthermore, although treatment was carried out by changing the time of administration or concentration in this experiment, there was no obvious difference in the number of flower setting. No obvious difference in the number of flower setting can be explained by communication among individuals via a pheromone.

In 1937, Mikhail Chailakhyan from former Soviet Union proposed the existence of a substance that is synthesized in the leaf and subsequently translocated to the shoot to induce floral bud formation. This substance is called a flowering hormone. Although this substance was not able to be identified over many years, presently, FT protein is considered to be the principal body of this flowering hormone from various studies. This hypothesis, however, is only a presumption from the results obtained in various studies. In order to eventually prove that FT protein is a flowering hormone, FT protein needs to be injected into a plant body to induce flowering. By this experiment, the present inventors eventually proved that FT protein is a flowering hormone. Moreover, the present inventors applied FT protein to agriculture for the first time. In addition, they proposed the existence of a flowering pheromone.

In the above-described examples, administration of a flowering inducer of the present invention into *Citrus unshiu* was described as an exemplary case. As will be described later, a flowering inducer of the present invention is also applicable to other plants.

First, application of a flowering inducer of the present invention to other citruses will be described. Since the amino acid sequence of CiFT2 protein has 99% identity with that of trifoliate orange FT protein, the amino acid sequence of FT protein is assumed to be fairly well conserved among citruses. Therefore, according to a preferable embodiment of the present invention, instead of using FT protein derived from a certain citrus species, CiFT2 protein derived from *Citrus unshiu* can be used to induce flowering of that certain citrus species.

Next, application of a flowering inducer of the present invention to other fruit trees will be described. CiFT2 protein shows an identity of 80% or more with a known fruit trees-derived FT protein at an amino acid sequence level. According to a preferable embodiment of the present invention, since *Citrus unshiu*-derived CiFT2 protein shows such high homology, this protein can be administered to other fruit tree to induce flowering. According to another preferable embodiment of the present invention, recombinant FT protein derived from a fruit tree to be administered can also be used. According to another preferable embodiment of the present invention, FT protein derived from other plant such as CiFT2 protein can also be used for inducing flowering. This is based on a report saying that flowering was accelerated in gene recombinant *Arabidopsis* that highly expressed *Citrus unshiu-*derived CiFT gene (Yasushi Kobayashi, Hidetaka Kaya, Koji Goto, Masaki Iwabuchi, and Takashi Araki (1999) "A Pair of Related Genes with Antagonistic Roles in Mediating Flowering Signals" Science 286, 1960-1962.), and thus the flowering-inducing function of FT protein seems to be less specific to species.

### EXAMPLE 2

### 6. Administration of flowering inducer and results therefrom (No.2)

### 6. 1. Administration of flowering inducer

*Citrus unshiu* nucellar seedling was grafted in two stages to a tip of a one-year-old seedling (obtained by grafting *Citrus unshiu* scion at the mature stage to a trifoliate orange rootstock), to use the resultant to carry out an experiment of injecting FT protein. Specifically, a trifoliate orange was used as a rootstock, *Citrus unshiu* at the mature stage was used as an intermediate rootstock, and *Citrus unshiu* nucellar seedling was used as a scion. A nucellar seedling refers to a shoot that arose from a nucellar embryo and not a shoot that arose from a fertilized embryo. A nucellar seedling is one type of products resulting from asexual reproduction and is a parent clone. Incidentally, a seedling refers to a growth that develops a shoot from a seed (independent from grafting/cuttage). Such a seedling occurs at the juvenile stage.

To explain the concept here, whether or not a part of the scion is derived from a nucellar embryo is not important, but that the part of scion is a seedling, in other words, the part of the scion is in the juvenile stage, is important.

Injection of a flowering inducer was performed in September, October and November, 2008. Injection was performed on *Citrus unshiu* in the mature stage as the intermediate rootstock, but not on the *Citrus unshiu* nucellar seedling as the scion. Specifically, injection was performed on the upper part of the site on the main stem where the scion of *Citrus unshiu* in the mature stage was grafted to the trifoliate orange rootstock. The recombinant CiFT2 protein obtained in Example 1 was diluted in a phosphate buffered saline to prepare a 500 ppm flowering inducer. One ml of 500 ppm (0.5 mg/ml) flowering inducer was drawn into a plastic dropper. Then, the epidermis was cut to the xylem, into which the tip of the plastic dropper was inserted where the inserted region was covered by an adhesive (Aron Alpha^{™} (Toagosei)) so as to prevent leaking of the liquid of the flowering inducer. The dropper was secured to the stalk using a tape. Note that the liquid was not injected by pressing the dropper but by natural absorption.

As a control, a plant was used that was not treated with the flowering inducer and that was placed in a land section (control section) 2.9 m away from the land section of the plant treated with the flowering inducer (FT-treated section).

### 6.2. Results from flowering inducer administration

The results from the above-described experiment of administering a flowering inducer are shown in Figure 13. The numerical data in Figure 13 are summarized in Table 5. As can be appreciated from Figure 13 and Table 5, there is no difference in the number of flower setting in the group administered with the flowering inducer (NS-Sep-1-NS-Nov-3) from the group placed in the control section (Cont-1-3).

**[Table 5]**

| | | | |
|---|---|---|---|
| | Leafy flower | Leafless flower | Vegetative shoot |

| | (Flowering shoot) | | (Non-flowering shoot) |
|---|---|---|---|
| cont-3 | 0 | 0 | 55 |
| cont-2 | 26 | 10 | 40 |
| cont-1 | 20 | 4 | 27 |
| NS-Nov-3 | 16 | 7 | 29 |
| NS-Nov-2 | 31 | 5 | 42 |
| NS-Nov-1 | 0 | 0 | 35 |
| NS-Oct-4 | 26 | 9 | 31 |
| NS-Oct-3 | 21 | 11 | 19 |
| NS-Oct-2 | 37 | 23 | 18 |
| NS-Oct-1 | 17 | 0 | 35 |
| NS-Sep-3 | 40 | 23 | 12 |
| NS-Sep-2 | 0 | 0 | 43 |
| NS-Sep-1 | 0 | 0 | 32 |

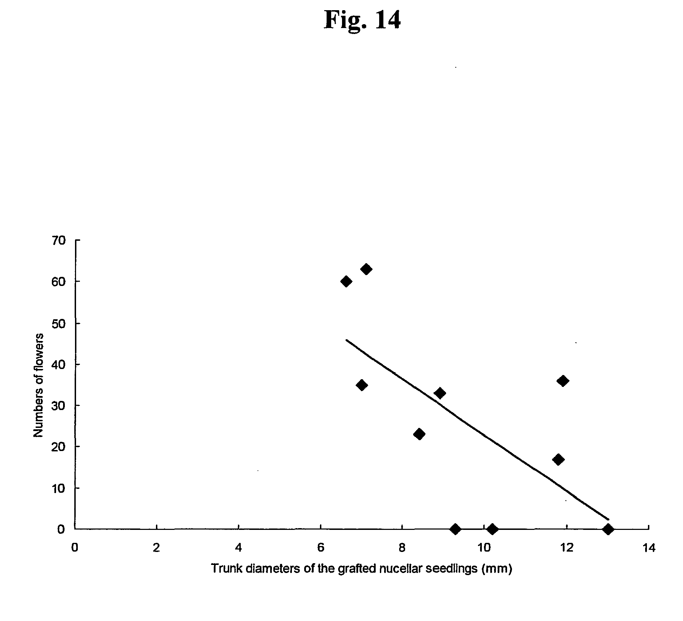

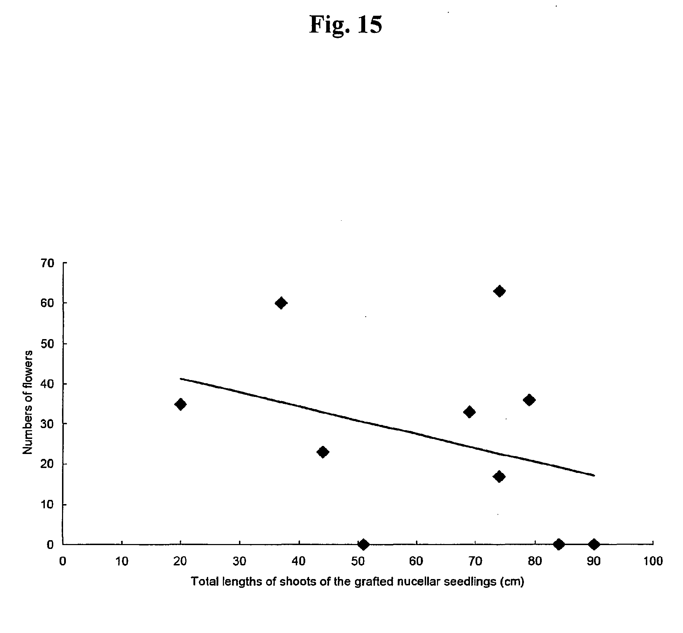

Furthermore, for the group administered with the flowering inducer (NS-Sep-1-NS-Nov-3), the presence of correlation between the stem diameter of the nucellar seedling and the number of flower setting was examined, and found that there was a negative correlation between the stem diameter of the nucellar seedling and the number of flower setting (Figure 14). Meanwhile, there was no correlation between the height of the nucellar seedling and the number of flower setting (Figure 15).

Hence, the followings can be implicated by the above results.
1) A substance that suppresses the action of FT protein is released from the nucellar seedling.
2) This substance translocates from the nucellar seedling to the intermediate rootstock and thereby suppresses the action of FT protein.
3) The larger the stem diameter (as an indication of growth) of the nucellar seedling, the greater the amount of this substance.

In addition, other experiments were also performed. A nucellar seedling of *Citrus natsudaidai Hayata f.Kawanonatsudaidai* or a naturally pollinated hybrid seedling *of Kawachi bankan* (Citrus grandis) was grown to obtain a seedling three months after the germination. FT protein was injected to them but they did not result in flowering even after a year. From these results, the seedling is suggested to have a substance that suppresses the action of FT protein.

Accordingly, since *Citrus unshiu* at the nutritional mature stage differs from those at the juvenile stage such as seedling and expected to have less amount of the substance that suppresses FT protein, the flowering inducing effect resulting from the flowering inducer can be stronger for those at the nutritional mature stage.

### SEQUENCE LISTING

[SEQ ID NO:1] A nucleotide sequence of DNA obtained in the example.
[SEQ ID NO:2] An amino acid sequence deduced from the nucleotide sequence represented by SEQ ID NO:1.
[SEQ ID NO:3] DNA sequence having DNA coding for a thrombin recognition site, DNA coding for a T7 tag and DNA coding for a histidine tag fused in this order following DNA consisting of the nucleotide sequence represented by SEQ ID NO:1.
[SEQ ID NO:4] An amino acid sequence deduced from the nucleotide sequence represented by SEQ ID NO:3.
[SEQ ID NO:5] cDNA sequence of *Citrus unshiu* CiFT (Accession No. AB027456).
[SEQ ID NO:6] An amino acid sequence deduced from the nucleotide sequence represented by SEQ ID NO:5.
[SEQ ID NO:7] cDNA sequence of *Citrus unshiu* CiFT2 (Accession No. AB301934).
[SEQ ID NO:8] An amino acid sequence deduced from the nucleotide sequence represented by SEQ ID NO:7.
[SEQ ID NO:9] Primer
[SEQ ID NO:10] Primer
[SEQ ID NO:11] Primer
[SEQ ID NO:12] Primer
[SEQ ID NO:13] Primer
[SEQ ID NO:14] Primer
[SEQ ID NO:15] Primer

## Claims

1. A method for inducing flowering comprising the step of injecting into a plant body
(i) any one of proteins (A)-(C) below:
(A) a purified protein comprising the amino acid sequence represented by SEQ ID NO:2;
(B) a purified protein comprising an amino acid sequence having deletion, substitution and/or addition of one or several amino acids in the amino acid sequence represented by SEQ ID NO:2 and having a flowering-inducing activity; and
(C) a purified protein comprising an amino acid sequence having an identity of 60% or more with the amino acid sequence represented by SEQ ID NO:2 and having a flowering-inducing activity; or
(ii) a purified protein encoded by a gene comprising any one of DNAs (a)-(c) below:
(a) DNA consisting of the nucleotide sequence represented by SEQ ID NO:1
(b) DNA that hybridizes with DNA consisting of a nucleotide sequence complementary to the nucleotide sequence represented by SEQ ID NO:1 under stringent conditions and that codes for a protein having a flowering-inducing activity; and
(c) DNA comprising a nucleotide sequence having an identity of 60% or more with the nucleotide sequence represented by SEQ ID NO:1 and coding for a protein having a flowering-inducing activity.

2. The method according to Claim 1, wherein the said purified protein (C) comprises an amino acid sequence having a said sequence identity of 95% or more.

3. The method according to Claim 1, wherein the said purified protein (ii) is encoded by a gene comprising a said DNA (c) which comprises a nucleotide sequence having a said sequence identity of 95% or more.

4. The method according to Claim 1, wherein the protein injected into the plant body is the purified protein consisting of the amino acid sequence represented by SEQ ID NO:4.

5. The method according to Claim 1, wherein the protein injected into the plant body is the purified protein encoded by a gene comprising DNA consisting of the nucleotide sequence represented by SEQ ID NO:3.

6. The method according to any one of Claims 1 to 5, wherein the plant is a fruit tree.

7. The method according Claim 6, wherein the fruit tree is citrus.

8. The method according to Claim 7, wherein the citrus is *Citrus unshiu.*

9. The method according to any one of Claims 1 to 8, wherein the plant is a plant obtained by grafting a scion at the mature stage to a rootstock.

10. The method according to any one of Claims 1 to 9, wherein the plant treated with the protein is placed next to a plant targeted for flowering induction that has not been treated with the protein.

11. Use of a purified protein as defined in any one of Claims 1 to 5 for inducing flowering of a plant by injecting the said purified protein into the plant body.

12. The use according to Claim 11, wherein the plant is as defined in any one of Claims 6 to 8.

13. The use according to Claim 11 or 12, wherein the plant is a plant obtained by grafting a scion at the mature stage to a rootstock.

14. The use according to any one of Claims 11 to 13, wherein the plant treated with the protein is placed next to a plant targeted for flowering induction that has not been treated with the protein.

## Patentansprüche

1. Verfahren zum Herbeiführen der Blüte aufweisend den Schritt des Einspritzens in einen Pflanzenkörper
(i) irgendeines der Proteine (A) - (C) unten:
(A) ein gereinigtes Protein, das die Aminosäuresequenz aufweist, die repräsentiert wird durch SEQ ID NO:2;
(B) ein gereinigtes Protein, das eine Aminosäuresequenz aufweist, die eine Deletion, Substitution und/oderAddition einer oder mehrererAminosäuren in der Aminosäuresequenz, die repräsentiert wird durch SEQ ID NO:2, hat und eine die Blüte herbeiführende Aktivität hat;
(C) ein gereinigtes Protein, das eine Aminosäuresequenz aufweist, die eine Übereinstimmung von 60 % oder mehr mit der Aminosäuresequenz, die repräsentiert wird durch SEQ ID NO:2, hat und eine die Blüte herbeiführende Aktivität hat; oder
(ii) eines gereinigten Proteins, das kodiert wird von einem Gen, das irgendeine der DNAs (a) - (c) unten aufweist:
(a) DNA, die aus der Nucleotidsequenz besteht, die repräsentiert wird durch SEQ ID NO:1;
(b) DNA, die unter stringenten Bedingungen mit DNA, die aus einer Nucleotidsequenz besteht, die komplementär ist zu der Nucleotidsequenz, die repräsentiert wird durch SEQ ID NO:1, hybridisiert und die für ein Protein mit einer die Blüte herbeiführenden Aktivität kodiert; und
(c) DNA, die eine Nucleotidsequenz aufweist, die eine Übereinstimmung von 60 % oder mehr mit der Nucleotidsequenz hat, die repräsentiert wird durch SEQ ID NO:1, und die für ein Protein mit einer die Blüte herbeiführenden Aktivität kodiert.

2. Verfahren nach Anspruch 1, wobei das gereinigte Protein (C) eine Aminosäuresequenz aufweist, bei der die Sequenzübereinstimmung 95 % oder mehr beträgt.

3. Verfahren nach Anspruch 1, wobei das gereinigte Protein (ii) kodiert wird von einem Gen, das die DNA (c) aufweist, die eine Nucleotidsequenz aufweist, bei der die Sequenzübereinstimmung 95 % oder mehr beträgt.

4. Verfahren nach Anspruch 1, wobei das in den Pflanzenkörper eingespritzte Protein das gereinigte Protein ist, das aus der Aminosäuresequenz besteht, die repräsentiert wird durch SEQ ID NO:4.

5. Verfahren nach Anspruch 1, wobei das in den Pflanzenkörper eingespritzte Protein das gereinigte Protein ist, das von einem Gen kodiert wird, das DNA aufweist, die aus der Nucleotidsequenz besteht, die repräsentiert wird durch SEQ ID NO:3.

6. Verfahren nach einem der Ansprüche 1 bis 5, wobei die Pflanze ein Obstbaum ist.

7. Verfahren nach Anspruch 6, wobei der Obstbaum ein Zitrusbaum ist.

8. Verfahren nach Anspruch 7, wobei der Zitrusbaum Satsuma ist.

9. Verfahren nach einem der Ansprüche 1 bis 8, wobei die Pflanze eine Pflanze ist, die erhalten wurde durch Pfropfen eines Reises im Reifestadium auf einen Wurzelstock.

10. Verfahren nach einem der Ansprüche 1 bis 9, wobei die mit dem Protein behandelte Pflanze nahe an einer Pflanze, bei der die Blüte herbeigeführt werden soll, die nicht mit dem Protein behandelt wurde, platziert wird.

11. Verwendung eines gereinigten Proteins, wie es in einem der Ansprüche 1 bis 5 definiert ist, zum Herbeiführen der Blüte einer Pflanze durch Einspritzen des gereinigten Proteins in den Pflanzenkörper.

12. Verwendung nach Anspruch 11, wobei die Pflanze so ist, wie in einem der Ansprüche 6 bis 8 definiert.

13. Verwendung nach Anspruch 11 oder 12, wobei die Pflanze eine Pflanze ist, die erhalten wurde durch Pfropfen eines Reises im Reifestadium auf einen Wurzelstock.

14. Verwendung nach einem der Ansprüche 11 bis 13, wobei die mit dem Protein behandelte Pflanze nahe an einer Pflanze, bei der die Blüte herbeigeführt werden soll, die nicht mit dem Protein behandelt wurde, platziert wird.

## Revendications

1. Procédé pour induire la floraison comprenant l'étape d'injection dans un corps d'une plante
(i) de l'une quelconque des protéines (A)-(C) ci-dessous:
(A) une protéine purifiée comprenant la séquence d'amino-acides représentée par SEQ ID NO:2 ;
(B) une protéine purifiée comprenant une séquence d'amino-acides ayant une délétion, substitution et/ou addition d'un ou plusieurs aminoacides dans la séquence d'aminoacides représentée par SEQ ID NO:2 et ayant une activité induisant la floraison ; et
(C) une protéine purifiée comprenant une séquence d'amino-acides ayant une identité de 60 % ou plus avec la séquence d'aminoacides représentée par SEQ ID NO:2 et ayant une activité induisant la floraison ; ou
(ii) une protéine purifiée codée par un gène comprenant l'un quelconque des ADN (a)-(c) ci-dessous :
(a) un ADN consistant en la séquence nucléotidique représentée par SEQ ID NO:1 ;
(b) un ADN qui s'hybride avec un ADN consistant en une séquence nucléotidique complémentaire de la séquence nucléotidique représentée par SEQ ID NO:1 dans des conditions stringentes et qui code une protéine ayant une activité induisant la floraison ; et
(c) un ADN comprenant une séquence nucléotidique ayant une identité de 60 % ou plus avec la séquence nucléotidique représentée par SEQ ID NO:1 et codant une protéine ayant une activité induisant la floraison.

2. Procédé selon la revendication 1, où ladite protéine purifiée (C) comprend une séquence d'aminoacides ayant une dite identité de séquence de 95 % ou plus.

3. Procédé selon la revendication 1, où ladite protéine purifiée (ii) est codée par un gène comprenant un dit ADN (c) qui comprend une séquence nucléotidique ayant une dite identité de séquence de 95 % ou plus.

4. Procédé selon la revendication 1, où la protéine injectée dans le corps d'une plante est la protéine purifiée consistant en la séquence d'aminoacides représentée par SEQ ID NO:4.

5. Procédé selon la revendication 1, où la protéine injectée dans le corps d'une plante est la protéine purifiée codée par un gène comprenant un ADN consistant en la séquence nucléotidique représentée par SEQ ID NO:3.

6. Procédé selon l'une quelconque des revendications 1 à 5, où la plante est un arbre fruitier.

7. Procédé selon la revendication 6, où l'arbre fruitier est un citronnier.

8. Procédé selon la revendication 7, où le citronnier est *Citrus unshiu.*

9. Procédé selon l'une quelconque des revendications 1 à 8, où la plante est une plante obtenue par greffage d'un scion au stade mature à une souche.

10. Procédé selon l'une quelconque des revendications 1 à 9, où la plante traitée avec la protéine est placée à proximité d'une plante ciblée pour l'induction de la floraison qui n'a pas été traitée avec la protéine.

11. Utilisation d'une protéine purifiée telle que définie dans l'une quelconque des revendications 1 à 5 pour induire la floraison d'une plante par injection de ladite protéine purifiée dans le corps de la plante.

12. Utilisation selon la revendication 11, où la plante est telle que définie dans l'une quelconque des revendications 6 à 8.

13. Utilisation selon la revendication 11 ou 12, où la plante est une plante obtenue par greffage d'un scion au stade mature à une souche.

14. Utilisation selon l'une quelconque des revendications 11 à 13, où la plante traitée avec la protéine est placée à proximité d'une plante ciblée pour l'induction de la floraison qui n'a pas été traitée avec la protéine.


### SEQUENCE LISTING

<110> Saga University
<120> Flowering inducer
<130> PCT10-0049
<150> JP 2009-260847
   <151> 2009-11-16
<160> 15
<170> PatentIn version 3.4
<210> 1
   <211> 531
   <212> DNA
   <213> Citrus unshiu
<220>
   <221> CDS
   <222> (1)..(531)
<400> 1
   atg tct agc agg gag aga gac cct ctt att gtt ggc cgc gtt gtt ggt      48
   Met Ser Ser Arg Glu Arg Asp Pro Leu Ile Val Gly Arg Val Val Gly
   1               5                   10                  15
   gat gtt ctt gac aat ttt aca aga aca att cca atg agg att acc tat      96
   Asp Val Leu Asp Asn Phe Thr Arg Thr Ile Pro Met Arg Ile Thr Tyr
               20                  25                  30
   tca aac aag gat gtt aat aat ggc cgt gag ctc aaa cct tct gaa gtt     144
   Ser Asn Lys Asp Val Asn Asn Gly Arg Glu Leu Lys Pro Ser Glu Val
           35                  40                  45
   ctg aac cag cct agg gtt gaa att ggt ggt gat gat ctt agg aca ttt     192
   Leu Asn Gln Pro Arg Val Glu Ile Gly Gly Asp Asp Leu Arg Thr Phe
       50                  55                  60
   tat act ttg gta atg gtt gat cct gat gca cca agc cca agt gac ccc     240
   Tyr Thr Leu Val Met Val Asp Pro Asp Ala Pro Ser Pro Ser Asp Pro
   65                  70                  75                  80
   agc ctt agg gag tat ttg cat tgg ttg gtg act gat att cca gca acc     288
   Ser Leu Arg Glu Tyr Leu His Trp Leu Val Thr Asp Ile Pro Ala Thr
                   85                  90                  95
   aca ggg gcc agc ttt ggc caa gag att gtg aac tat gaa agc cct agt     336
   Thr Gly Ala Ser Phe Gly Gln Glu Ile Val Asn Tyr Glu Ser Pro Ser
               100                 105                 110
   cca acg atg ggg att cac agg ttt gtc ttt gtg ttg ttc cgg caa ctt     384
   Pro Thr Met Gly Ile His Arg Phe Val Phe Val Leu Phe Arg Gln Leu
           115                 120                 125
   ggg agg cag act gtt tat gca cca ggg tgg cgt cag aac ttc agc acg     432
   Gly Arg Gln Thr Val Tyr Ala Pro Gly Trp Arg Gln Asn Phe Ser Thr
       130                 135                 140
   agg gat ttt gct gag ctt tac aat ctg gga cct ccg gtg gcc gct gtc     480
   Arg Asp Phe Ala Glu Leu Tyr Asn Leu Gly Pro Pro Val Ala Ala Val
   145                 150                 155                 160
   tac ttc aac tgc cag agg gag agc gga tcc ggc gga agg cct gtc aga     528
   Tyr Phe Asn Cys Gln Arg Glu Ser Gly Ser Gly Gly Arg Pro Val Arg
                   165                 170                 175
   cga                                                                 531
   Arg
<210> 2
   <211> 177
   <212> PRT
   <213> Citrus unshiu
<400> 2
   Met Ser Ser Arg Glu Arg Asp Pro Leu Ile Val Gly Arg Val Val Gly
   1               5                   10                  15
   Asp Val Leu Asp Asn Phe Thr Arg Thr Ile Pro Met Arg Ile Thr Tyr
               20                  25                  30
   Ser Asn Lys Asp Val Asn Asn Gly Arg Glu Leu Lys Pro Ser Glu Val
           35                  40                  45
   Leu Asn Gln Pro Arg Val Glu Ile Gly Gly Asp Asp Leu Arg Thr Phe
       50                  55                  60
   Tyr Thr Leu Val Met Val Asp Pro Asp Ala Pro Ser Pro Ser Asp Pro
   65                  70                  75                  80
   Ser Leu Arg Glu Tyr Leu His Trp Leu Val Thr Asp Ile Pro Ala Thr
                   85                  90                  95
   Thr Gly Ala Ser Phe Gly Gln Glu Ile Val Asn Tyr Glu Ser Pro Ser
               100                 105                 110
   Pro Thr Met Gly Ile His Arg Phe Val Phe Val Leu Phe Arg Gln Leu
           115                 120                 125
   Gly Arg Gln Thr Val Tyr Ala Pro Gly Trp Arg Gln Asn Phe Ser Thr
       130                 135                 140
   Arg Asp Phe Ala Glu Leu Tyr Asn Leu Gly Pro Pro Val Ala Ala Val
   145                 150                 155                 160
   Tyr Phe Asn Cys Gln Arg Glu Ser Gly Ser Gly Gly Arg Pro Val Arg
                   165                 170                 175
   Arg
<210> 3
   <211> 660
   <212> DNA
   <213> Artificial sequence
<220>
   <223> synthetic DNA
<220>
   <221> CDS
   <222> (1)..(660)
<400> 3
   atg tct agc agg gag aga gac cct ctt att gtt ggc cgc gtt gtt ggt 48
   Met Ser Ser Arg Glu Arg Asp Pro Leu Ile Val Gly Arg Val Val Gly
   1 5 10 15
   gat gtt ctt gac aat ttt aca aga aca att cca atg agg att acc tat 96
   Asp Val Leu Asp Asn Phe Thr Arg Thr Ile Pro Met Arg Ile Thr Tyr
   20 25 30
   tca aac aag gat gtt aat aat ggc cgt gag ctc aaa cct tct gaa gtt 144
   Ser Asn Lys Asp Val Asn Asn Gly Arg Glu Leu Lys Pro Ser Glu Val
   35 40 45
   ctg aac cag cct agg gtt gaa att ggt ggt gat gat ctt agg aca ttt 192
   Leu Asn Gln Pro Arg Val Glu Ile Gly Gly Asp Asp Leu Arg Thr Phe
   50 55 60
   tat act ttg gta atg gtt gat cct gat gca cca agc cca agt gac ccc 240
   Tyr Thr Leu Val Met Val Asp Pro Asp Ala Pro Ser Pro Ser Asp Pro
   65 70 75 80
   agc ctt agg gag tat ttg cat tgg ttg gtg act gat att cca gca acc 288
   Ser Leu Arg Glu Tyr Leu His Trp Leu Val Thr Asp Ile Pro Ala Thr
   85 90 95
   aca ggg gcc agc ttt ggc caa gag att gtg aac tat gaa agc cct agt 336
   Thr Gly Ala Ser Phe Gly Gln Glu Ile Val Asn Tyr Glu Ser Pro Ser
   100 105 110
   cca acg atg ggg att cac agg ttt gtc ttt gtg ttg ttc cgg caa ctt 384
   Pro Thr Met Gly Ile His Arg Phe Val Phe Val Leu Phe Arg Gln Leu
   115 120 125
   ggg agg cag act gtt tat gca cca ggg tgg cgt cag aac ttc agc acg 432
   Gly Arg Gln Thr Val Tyr Ala Pro Gly Trp Arg Gln Asn Phe Ser Thr
   130 135 140
   agg gat ttt gct gag ctt tac aat ctg gga cct ccg gtg gcc gct gtc 480
   Arg Asp Phe Ala Glu Leu Tyr Asn Leu Gly Pro Pro Val Ala Ala Val
   145 150 155 160
   tac ttc aac tgc cag agg gag agc gga tcc ggc gga agg cct gtc aga 528
   Tyr Phe Asn Cys Gln Arg Glu Ser Gly Ser Gly Gly Arg Pro Val Arg
   165 170 175
   cga agc agc ggc ctg gtg ccg cgc ggc agc cat atg gct agc atg act 576
   Arg Ser Ser Gly Leu Val Pro Arg Gly Ser His Met Ala Ser Met Thr
   180 185 190
   ggt gga cag caa atg ggt cgg gat ccg aat tcg agc tcc gtc gac aag 624
   Gly Gly Gln Gln Met Gly Arg Asp Pro Asn Ser Ser Ser Val Asp Lys
   195 200 205
   ctt gcg gcc gca ctc gag cac cac cac cac cac cac 660
   Leu Ala Ala Ala Leu Glu His His His His His His
   210 215 220
<210> 4
   <211> 220
   <212> PRT
   <213> Artificial sequence
<220>
   <223> Synthetic Construct
<400> 4
   Met Ser Ser Arg Glu Arg Asp Pro Leu Ile Val Gly Arg Val Val Gly
   1               5                   10                  15
   Asp Val Leu Asp Asn Phe Thr Arg Thr Ile Pro Met Arg Ile Thr Tyr
               20                  25                  30
   Ser Asn Lys Asp Val Asn Asn Gly Arg Glu Leu Lys Pro Ser Glu Val
           35                  40                  45
   Leu Asn Gln Pro Arg Val Glu Ile Gly Gly Asp Asp Leu Arg Thr Phe
       50                  55                  60
   Tyr Thr Leu Val Met Val Asp Pro Asp Ala Pro Ser Pro Ser Asp Pro
   65                  70                  75                  80
   Ser Leu Arg Glu Tyr Leu His Trp Leu Val Thr Asp Ile Pro Ala Thr
                   85                  90                  95
   Thr Gly Ala Ser Phe Gly Gln Glu Ile Val Asn Tyr Glu Ser Pro Ser
               100                 105                 110
   Pro Thr Met Gly Ile His Arg Phe Val Phe Val Leu Phe Arg Gln Leu
           115                 120                 125
   Gly Arg Gln Thr Val Tyr Ala Pro Gly Trp Arg Gln Asn Phe Ser Thr
       130                 135                 140
   Arg Asp Phe Ala Glu Leu Tyr Asn Leu Gly Pro Pro Val Ala Ala Val
   145                 150                 155                 160
   Tyr Phe Asn Cys Gln Arg Glu Ser Gly Ser Gly Gly Arg Pro Val Arg
                   165                 170                 175
   Arg Ser Ser Gly Leu Val Pro Arg Gly Ser His Met Ala Ser Met Thr
               180                 185                 190
   Gly Gly Gln Gln Met Gly Arg Asp Pro Asn Ser Ser Ser Val Asp Lys
                 195                 200                 205
   Leu Ala Ala Ala Leu Glu His His His His His His
       210                 215                 220
<210> 5
   <211> 745
   <212> DNA
   <213> Citrus unshiu
<220>
   <221> CDS
   <222> (96) .. (629)
<400> 5
   ggcacgagga atagtcttac tacttttgta ggctgtgtgt gtatttgttt gtgcttagtg      60
   ttgttgagtg tttgtttgtg tttagtgttg ttgat atg tct agc agg gag aga        113
                                          Met Ser Ser Arg Glu Arg
                                          1               5
   gat cct ctt att gtt ggc cgc gtt gtt ggt gat gtt ctt gac aat ttt       161
   Asp Pro Leu Ile Val Gly Arg Val Val Gly Asp Val Leu Asp Asn Phe
               10                  15                  20
   aca aga aca att cca atg agg att acc tat tca aac aag gat gtt aat       209
   Thr Arg Thr Ile Pro Met Arg Ile Thr Tyr Ser Asn Lys Asp Val Asn
           25                  30                  35
   aat ggc cgt gag ctc aaa cct tct gaa gtt ctg aac cag cct agg gct       257
   Asn Gly Arg Glu Leu Lys Pro Ser Glu Val Leu Asn Gln Pro Arg Ala
       40                  45                  50
   gaa att ggt ggt gat gat ctt agg aca ttt tat act ttg gta atg gtt       305
   Glu Ile Gly Gly Asp Asp Leu Arg Thr Phe Tyr Thr Leu Val Met Val
   55                  60                  65                  70
   gat cct gat gca cca agc cca agt gac ccc agc ctt agg gag tat ttg       353
   Asp Pro Asp Ala Pro Ser Pro Ser Asp Pro Ser Leu Arg Glu Tyr Leu
                   75                  80                  85
   cat tgg ttg gtg act gat att cca gca acc aca ggg gcc agc ttt ggc       401
   His Trp Leu Val Thr Asp Ile Pro Ala Thr Thr Gly Ala Ser Phe Gly
               90                  95                  100
   caa gag att gtg aac tat gaa agc cct agg cca acg atg ggg att cac       449
   Gln Glu Ile Val Asn Tyr Glu Ser Pro Arg Pro Thr Met Gly Ile His
           105                 110                 115
   agg ttt gtc ttt gtg ttg ttc cgg caa ctt ggg agg cag act gtt tat       497
   Arg Phe Val Phe Val Leu Phe Arg Gln Leu Gly Arg Gln Thr Val Tyr
       120                 125                 130
   gca cca ggg tgg cgt cag aac ttc agc acg agg gat ttt gct gag ctt       545
   Ala Pro Gly Trp Arg Gln Asn Phe Ser Thr Arg Asp Phe Ala Glu Leu
   135                 140                 145                 150
   tac aat ctg gga cct ccg gtg gcc gct gtc tac ttc aac tgc cag agg       593
   Tyr Asn Leu Gly Pro Pro Val Ala Ala Val Tyr Phe Asn Cys Gln Arg
                   155                 160                 165
   gag agc gga tcc ggc gga agg cct gtc aga cga tga tccatacatg            639
   Glu Ser Gly Ser Gly Gly Arg Pro Val Arg Arg
               170                 175
   cttaatttga tatcaaatta cacacacaca cacacacaca cacacacaca cacacacaca    699
   cacacacaca cactatttat atatatatat atatatatat atatat                   745
<210> 6
   <211> 177
   <212> PRT
   <213> Citrus unshiu
<400> 6
   Met Ser Ser Arg Glu Arg Asp Pro Leu Ile Val Gly Arg Val Val Gly
   1               5                   10                  15
   Asp Val Leu Asp Asn Phe Thr Arg Thr Ile Pro Met Arg Ile Thr Tyr
               20                  25                  30
   Ser Asn Lys Asp Val Asn Asn Gly Arg Glu Leu Lys Pro Ser Glu Val
           35                  40                  45
   Leu Asn Gln Pro Arg Ala Glu Ile Gly Gly Asp Asp Leu Arg Thr Phe
       50                  55                  60
   Tyr Thr Leu Val Met Val Asp Pro Asp Ala Pro Ser Pro Ser Asp Pro
   65                  70                  75                  80
   Ser Leu Arg Glu Tyr Leu His Trp Leu Val Thr Asp Ile Pro Ala Thr
                   85                  90                  95
   Thr Gly Ala Ser Phe Gly Gln Glu Ile Val Asn Tyr Glu Ser Pro Arg
               100                 105                 110
   Pro Thr Met Gly Ile His Arg Phe Val Phe Val Leu Phe Arg Gln Leu
           115                 120                 125
   Gly Arg Gln Thr Val Tyr Ala Pro Gly Trp Arg Gln Asn Phe Ser Thr
       130                 135                 140
   Arg Asp Phe Ala Glu Leu Tyr Asn Leu Gly Pro Pro Val Ala Ala Val
   145                 150                 155                 160
   Tyr Phe Asn Cys Gln Arg Glu Ser Gly Ser Gly Gly Arg Pro Val Arg
                   165                 170                 175
   Arg
<210> 7
   <211> 946
   <212> DNA
   <213> Citrus unshiu
<220>
   <221> CDS
   <222> (117)..(650)
<400> 7
   tccaacctac caacaaaatt tcatcacttg aatagtctta ctacttttgt aggttgtttg      60
   tgtatttgtt tgtgcttagt gttgttgagt gtttgtttgt gtttagtgtt gttgat atg     119
                                                                 Met
                                                                 1
   tct agc agg gag aga gac cct ctt att gtt ggc cgc gtt gtt ggt gat       167
   Ser Ser Arg Glu Arg Asp Pro Leu Ile Val Gly Arg Val Val Gly Asp
               5                   10                  15
   gtt ctt gac aat ttt aca aga aca att cca atg agg att acc tat tca       215
   Val Leu Asp Asn Phe Thr Arg Thr Ile Pro Met Arg Ile Thr Tyr Ser
           20                  25                  30
   aac aag gat gtt aat aat ggc cgt gag ctc aaa cct tct gaa gtt ctg       263
   Asn Lys Asp Val Asn Asn Gly Arg Glu Leu Lys Pro Ser Glu Val Leu
       35                  40                  45
   aac cag cct agg gtt gaa att ggt ggt gat gat ctt agg aca ttt tat       311
   Asn Gln Pro Arg Val Glu Ile Gly Gly Asp Asp Leu Arg Thr Phe Tyr
   50                  55                  60                  65
   act ttg gta atg gtt gat cct gat gca cca agc cca agt gac ccc agc       359
   Thr Leu Val Met Val Asp Pro Asp Ala Pro Ser Pro Ser Asp Pro Ser
                   70                  75                  80
   ctt agg gag tat ttg cat tgg ttg gtg act gat att cca gca acc aca       407
   Leu Arg Glu Tyr Leu His Trp Leu Val Thr Asp Ile Pro Ala Thr Thr
               85                  90                  95
   ggg gcc agc ttt ggc caa gag att gtg aac tat gaa agc cct agt cca       455
   Gly Ala Ser Phe Gly Gln Glu Ile Val Asn Tyr Glu Ser Pro Ser Pro
           100                 105                 110
   acg atg ggg att cac agg ttt gtc ttt gtg ttg ttc cgg caa ctt ggg       503
   Thr Met Gly Ile His Arg Phe Val Phe Val Leu Phe Arg Gln Leu Gly
       115                 120                 125
   agg cag act gtt tat gca cca ggg tgg cgt cag aac ttc agc acg agg       551
   Arg Gln Thr Val Tyr Ala Pro Gly Trp Arg Gln Asn Phe Ser Thr Arg
   130                 135                 140                 145
   gat ttt gct gag ctt tac aat ctg gga cct ccg gtg gcc gct gtc tac       599
   Asp Phe Ala Glu Leu Tyr Asn Leu Gly Pro Pro Val Ala Ala Val Tyr
                   150                 155                 160
   ttc aac tgc cag agg gag agc gga tcc ggc gga agg cct gtc aga cga       647
   Phe Asn Cys Gln Arg Glu Ser Gly Ser Gly Gly Arg Pro Val Arg Arg
               165                 170                 175
   tga tccatacatg cttaatttga tatcaaatta cacacaaaca cacacacaca            700
   cacacactat atatatatat atccaaaatt aagagctagc tagtgttaac ataattatgt      760
   catggttaat cttcctctct ggctttctca cattttattg taacgtactc tgaagatgaa      820
   gttaaatact tcaaacagtg agaaaggaag attgttgtac tgtgtataca attaattaga      880
   ttgtaataaa aagagatgga attttttttt taaaaagcaa aaaaaaaaaa aaaaaaaaaa      940
   aaaaaa                                                                 946
<210> 8
   <211> 177
   <212> PRT
   <213> Citrus unshiu
<400> 8
   Met Ser Ser Arg Glu Arg Asp Pro Leu Ile Val Gly Arg Val Val Gly
   1               5                   10                  15
   Asp Val Leu Asp Asn Phe Thr Arg Thr Ile Pro Met Arg Ile Thr Tyr
               20                  25                  30
   Ser Asn Lys Asp Val Asn Asn Gly Arg Glu Leu Lys Pro Ser Glu Val
           35                  40                  45
   Leu Asn Gln Pro Arg Val Glu Ile Gly Gly Asp Asp Leu Arg Thr Phe
       50                  55                  60
   Tyr Thr Leu Val Met Val Asp Pro Asp Ala Pro Ser Pro Ser Asp Pro
   65                  70                  75                  80
   Ser Leu Arg Glu Tyr Leu His Trp Leu Val Thr Asp Ile Pro Ala Thr
                   85                  90                  95
   Thr Gly Ala Ser Phe Gly Gln Glu Ile Val Asn Tyr Glu Ser Pro Ser
               100                 105                 110
   Pro Thr Met Gly Ile His Arg Phe Val Phe Val Leu Phe Arg Gln Leu
           115                 120                 125
   Gly Arg Gln Thr Val Tyr Ala Pro Gly Trp Arg Gln Asn Phe Ser Thr
       130                 135                 140
   Arg Asp Phe Ala Glu Leu Tyr Asn Leu Gly Pro Pro Val Ala Ala Val
   145                 150                 155                 160
   Tyr Phe Asn Cys Gln Arg Glu Ser Gly Ser Gly Gly Arg Pro Val Arg
                   165                 170                 175
   Arg
<210> 9
   <211> 18
   <212> DNA
   <213> Artificial sequence
<220>
   <223> synthetic DNA
<400> 9
   caaattaagc atgtatgg 18
<210> 10
   <211> 18
   <212> DNA
   <213> Artificial sequence
<220>
   <223> synthetic DNA
<400> 10
   caaattaagc atgtatgg 18
<210> 11
   <211> 18
   <212> DNA
   <213> Artificial sequence
<220>
   <223> synthetic DNA
<400> 11
   gtgcttagtg ttgttgag 18
<210> 12
   <211> 32
   <212> DNA
   <213> Artificial sequence
<220>
   <223> synthetic DNA
<400> 12
   aggagatata ccatgtctag cagggagaga ga 32
<210> 13
   <211> 35
   <212> DNA
   <213> Artificial sequence
<220>
   <223> synthetic DNA
<400> 13
   caccaggccg ctgcttcgtc tgacaggcct tccgc 35
<210> 14
   <211> 35
   <212> DNA
   <213> Artificial sequence
<220>
   <223> synthetic DNA
<400> 14
   aattttgttt aactttaaga aggagatata ccatg 35
<210> 15
   <211> 35
   <212> DNA
   <213> Artificial sequence
<220>
   <223> synthetic DNA
<400> 15
   gccatatggc tgccgcgcgg caccaggccg ctgct 35